# EUROPÄISCHE PATENTSCHRIFT

(11) **EP 2 720 729 B1**
(45) Veröffentlichungstag und Bekanntmachung des Hinweises auf die Patenterteilung: **16.09.2015**
(21) Anmeldenummer: 12740875.5
(22) Anmeldetag: 19.06.2012
(51) Int. Cl.: A61M 1/02

(54) **VERFAHREN ZUR TRENNUNG VON BLUT, ABTRENNBEHÄLTER FÜR EINE BLUTZENTRIFUGE, SYSTEM ZUR BEFÜLLUNG EINES EINFRIERBEHÄLTERS**
METHOD FOR SEPARATING BLOOD, SEPARATION CONTAINER FOR A BLOOD CENTRIFUGE, SYSTEM FOR FILLING A FREEZER CONTAINER
PROCÉDÉ POUR LA SÉPARATION DU SANG, RÉCIPIENT DE SÉPARATION POUR UNE CENTRIFUGEUSE DE SANG, SYSTÈME POUR LE REMPLISSAGE D'UN RÉCIPIENT DE CONGÉLATION

(30) Priorität: 19.06.2011 DE 102011105311
(43) Veröffentlichungstag der Anmeldung: 23.04.2014
(73) Patentinhaber: Pobitschka, Walter, 61350 Bad Homburg (DE)
(72) Erfinder: Pobitschka, Walter, 61350 Bad Homburg (DE)
(74) Vertreter: Jendricke, Susann
(86) Internationale Anmeldenummer: PCT/DE2012/000628
(87) Internationale Veröffentlichungsnummer: WO 2012/175069

(56) Entgegenhaltungen:
- WO-A1-84/02091
- DE-A1- 2 741 398
- DE-A1-102008 047 068
- US-A- 4 040 959

## Beschreibung

Die Erfindung betrifft ein Verfahren zur Trennung von Blut, wobei verschiedene Blutfraktionen - Erythrozyten, Buffy-Coat und Blutplasma - gewonnen werden, wobei Blut in einen Abtrennbehälter eingefüllt wird und dann in verschiedene, übereinandergeordnete, strömungverbundene Abschnitte des Abtrennbehälters, nämlich Kopfabschnitt zur Aufnahme des Blutplasmas, Mittelabschnitt zur Aufnahme des Buffy-Coats, Fußabschnitt zur Aufnahme der Erythrozyten, zentrifugiert wird. Ein solches Verfahren wird zum Beispiel in WO84/02091 beschrieben.

Des weiteren betrifft die Erfindung einen Abtrennbehälter für eine Blutzentrifuge, insbesondere zur Durchführung des Verfahrens, mit einem Fußabschnitt zur Aufnahme von Erythrozyten, mit einem Mittelabschbnitt zur Aufnahme von Buffy-Coat und mit einem Kopfabschnitt zur Aufnahme von Blutplasma, wobei der Mittelabschnitt eine geringere Querschnittsabmessung aufweist als Kopf- und Fußabschnitt, wobei die Abschnitte strömungsverbunden sind und wobei der Fußabschnitt vom Mittelabschnitt abtrennbar ist.

Schließlich betrifft die Erfindung ein System zur Befüllung eines Einfrierbehälters mit einer nach Zentrifugierung gewonnenen Blutfraktion, nämliche Buffy-Coat, und zur Präparation des Buffy-Coats zum Zwecke der Kryokonservierung im Einfrierbehälter, insbesondere unter Verwendung einer Ausführungsform des erfindungsgemäßen Abtrennbehälters und insbesondere unter Durchführung einer Ausführungsform des erfindungsgemäßen Verfahrens.

Blut kann mittels einer Zentrifuge in verschiedene Blutfraktionen getrennt werden, indem die Zentrifuge bei bestimmten Umdrehungen und über eine bestimmte Zeitdauer schnell gedreht wird. Nach dem Zentrifugieren hat sich die Beschaffenheit des Blutes im Abtrennbehälter verändert. Die einzelnen Bestandteile haben sich aufgeteilt in Blutplasma, das vor allem aus Eiweiß und Wasser besteht, Buffy-Coat und Erythrozyten. Buffy-Coat enthält alle Leukozyten und den größten Teil an Thrombozyten. Die Erythrozyten weisen das höchste Gewicht auf, gefolgt von den Leukozyten und Thrombozyten im Buffy-Coat und dem Blutplasma.

Das Zerlegen von Blut in seine Bestandteile ist nicht nur bei Diagnoseverfahren oder anderen Laboranwendungen erforderlich, sondern auch im Bereich der Kryokonservierung von Nabelschnurblut. Das Einfrieren ist mit hohen Kosten verbunden. Der Platz in Tieftemperatureinrichtungen in Blutbanken ist begrenzt und teuer, da dort die Proben unter Anwendung von flüssigem Stickstoff tiefgefroren werden müssen. Insofern besteht ein allgemeines Bedürfnis nach Volumenreduktion. Das Einfriergut soll in möglichst kleinvolumigen Beuteln gelagert werden. Als Einfriergut ist Buffy-Coat von Interesse, da dort Stammzellen enthalten sind. Die abgetrennte Buffy-Coat-Fraktion muss haltbar gemacht werden, um sie später dem Patienten zuführen zu können. Die Haltbarmachung bzw. Kryokonservierung erfolgt unter Anwendung der chemischen Verbindung Dimethylsulfoxid, in weiteren DMSO genannt, in einem Einfrierbehälter. Die chemische Verbindung DMSO dient dazu, die Zellflüssigkeit im Präparat aufzukonzentrieren, um dort Unterschiede zwischen verschiedenen osmotischen Drücken auszugleichen. Hierdurch wird das Buffy-Coat bzw. die Zellsubstanz vor Destruktion / Platzen der Zellen geschützt. Alternativ zu reinem DMSO könnte auch ein vorgemischtes Kryokonservierungsmittel, wie bspw. DEXTRAN 40, zur Anwendung kommen.

Nach der Zentrifugierung hat sich zwischen der hier interessierende Buffy-Coat-Fraktion und den Erythrozyten eine Phasengrenze gebildet. Die Position der Phasengrenze ist je nach Bluteigenschaften und -menge immer anders. Dadurch wird die gezielte Entnahme des Buffy-Coats erschwert und die Ausbeute ist nicht optimal. Was die Grenze zwischen Blutplasma und Buffy-Cot angeht, so spielt diese eine untergeordnete Rolle. In der Regel wird sich das Blutplasma auch im Mittelabschnitt ausbreiten, da die Menge an Buffy-Coat sehr gering ist. Bei der Präparation der einzufrierenden Substanz wird Buffy-Coat gewöhnlich auch mit Blutplasma und weiteren Substanzen gemischt.

In DE 10 2008 047 068 A1 ist bereits eine Zentrifuge beschrieben, die über einen Abtrennbehälter mit zwei Behältnissen verfügt, die miteinander über einen flexiblen Kunststoff-Schlauch strömungsverbunden sind. Es sind ein Kopfabschnitt, ein Mittelabschnitt und ein Fußabschnitt vorhanden, in die die unterschiedlichen Fraktionen des Blutes zentrifugiert werden. Dort kommt es auf die Einhaltung geschlossener Systeme betreffend Zu- und Abführeinrichtungen und Abtrennbehälter und sterile Konnektierungen an. Das Buffy-Coat wird im Mittelabschnitt bzw. im Kunststoff-Schlauch gesammelt und der Mittelabschnitt kann über Klemmen vom Fußabschnitt, in dem sich die Erythrozyten sammeln, abgetrennt werden. Dort kann zwar das zu zentrifugierende Blut volumengenau zugegeben werden, jedoch wird bei dem in Rede stehenden Stand der Technik keine definierte Phasengrenze erreicht. Die Sammlung des Buffy-Coats im Mittelabschnitt, in den sich auch Blutplasma ausbreitet, geschieht nach Erfahrungswerten und das Volumen des Mittelabschnittes wird einmal mehr und einmal weniger ausgenutzt.

Ein Abtrennbehälter, der allerdings ausschließlich für das Sedimentationsverfahren vorgesehen ist, und auch drei Abschnitte - Kopfabschnitt, Mittelabschnitt mit gegenüber den beiden anderen Abschnitten geringem Querschnitt, Fußabschnitt - aufweist, ist aus DE 2741398 A1 bekannt. Dort wird beschrieben, dass Kopf- und Fußabschnitt ein Volumenverhältnis aufweisen, das im Wesentlichen dem Volumenverhältnis gleicht, das zwischen den Erythrozyten und dem Plasma im Blut herrscht. Die Oberfläche, die das Plasma von den Erythrozyten trennt, soll sich dann im Mittelteil bzw. in der dortigen Verbindungsleitung befinden. Wo genau die Phasengrenze verläuft, ist nicht beschrieben. Auch hier handelt es sich eher um Schätzungen und eine Konkretisierung der Phasengrenze im Hinblick auf unterschiedliche Blutproben- und -mengen ist nicht gegeben.

Der Erfindung liegt die Aufgabe zugrunde, ein Verfahren und einen Abtrennbehälter der in Rede stehenden Art sowie ein System anzugeben, wobei die Gewinnung von Buffy-Coat optimiert wird.

Mit dem Verfahren und dem Abtrennbehälter ist die Optimierung insbesondere auf den Erhalt einer definierte Phasengrenze zwischen den zentrifugierten Erythrozyten und dem zentrifugierten Buffy-Coat gerichtet, um die Ausbeute an Buffy-Coat zu optimieren.

Mit dem System ist die Optimierung auf Kontaminationsfreiheit ab dem Zeitpunkt, da das Blut in den Abtrennbehälter gelangt ist und den Erhalt eines handlichen Einfrierbehälters zur Kryokonservierung gerichtet, wodurch Gewinnung und darüber hinaus auch Transport und Lagerung des Buffy-Coats optimiert werden sollen. Mit dem System soll insbesondere ein zur Einlagerung / Kryokonservierung in einer Blutbank geeigneter Einfrierbehälter erzeugt werden, der mit Buffy-Coat unter kontaminationsfreien Bedingungen, unter Vermeidung des Kontaktes mit der Umwelt befüllt wurde. Insbesondere sollen mit dem System die entsprechenden, hier beschriebenen Verfahrensschritte ausgeführt werden unter Zuhilfenahme eines entsprechenden, hier beschriebenen Abtrennbehälters, in Bezug auf die Präparation mindestens eines Einfrierbehälters.

Die voranstehende Aufgabe wird im Hinblick auf das Verfahren durch die Merkmale des Patentanspruches 1 oder des Patentanspruchs 2 gelöst. Danach ist ein Verfahren der in Rede stehenden Art derart ausgestaltet, dass anhand des Hämatokritwertes des zugeführten Blutes das künftige Packvolumen der zu zentrifugierenden Erythrozyten bestimmt wird, dass der Fußabschnitt in seinem Aufnahmevolumen an das nach der Zentrifugierung zu erwartende Packvolumen der Erythrozyten derart angepasst wird, dass die sich beim Zentrifugieren ausbildende Phasengrenze zwischen Buffy-Coat und Erythrozyten in einem zum Fußabschnitt benachbarten Bereich des Mittelabschnittes des Abtrennbehälters positioniert wird, und dass die Menge an zugeführtem Blut volumengenau, unter Berücksichtigung des zu erwartenden Packvolumens der Erythrozyten, in den Abtrennbehälter eingegeben wird; ferner aufweisend die Merkmale des Kennzeichens des Anspruchs 1 bzw. 2.

Die voranstehende Aufgabe wird im Hinblick auf den Abtrennbehälter durch die Merkmale des Patentanspruches 9 oder 10 gelöst. Danach ist ein Abtrennbehälter der in Rede stehenden Art derart ausgestaltet, dass der Fußabschnitt bezüglich seines Aufnahmevolumens an das nach der Zentrifugierung zu erwartende Packvolumen der Erythrozyten so anpassbar ist, dass das zu erwartende Packvolumen vom Fußabschnitt im Wesentlichen vollständig aufnehmbar ist und dass hierdurch die Phasengrenze zwischen Erythrozyten und Buffy-Coat in einem zum Fußabschnitt benachbarten Bereich des Mittelabschnittes verläuft, ferner aufweisend die Merkmale des Kennzeichens des Ansrpruchs 9 bzw. 10.

Die voranstehende Aufgabe wird im Hinblick auf das System durch die Merkmale des Patentanspruches 21 gelöst. Danach weist das System folgende Merkmale auf:
- Es sind eine Zuführeinrichtung für Blut, ein Abtrennbehälter und ein Einfrierbehälter vorgesehen.
- Die Zuführeinrichtung für Blut umfasst mindestens eine Antikoagulans-Zuführung und einen Probenahmeabschnitt und ist mit dem Abtrennbehälter steril verbunden.
- Die Zuführeinrichtung für Blut ist nach der Befüllung des Abtrennbehälters und vor der Zentrifugierung vom Abtrennbehälter steril abtrennbar.
- Der Abtrennbehälter weist einen Fußabschnitt, einen Mittelabschnitt und einen Kopfabschnitt auf.
- Der Mittelabschnitt ist nach der Zentrifugierung unter Entfernung des Kopf- und Fußabschnittes zu einem Einfrierbehälter transformierbar.
- Der Mittelabschnitt bzw. der Einfrierbehälter ist mit einer Spritze, einer Zuführeinrichtung für DMSO und einer Druckausgleicheinrichtung steril verbunden.
- Die Spritze, die Zuführeinrichtung für DMSO und die Druckausgleicheinrichtung sind nach der Präparation des Buffy-Coats vom Abtrennbehälter steril abtrennbar, so, wie in Anspruch 21 definiert.

Ausgehend von dem aus der Praxis bekannten Stand der Technik ist hinsichtlich des Verfahrens erkannt worden, dass je nach Bluteigenschaften und -menge stets eine andere Position der Phasengrenze erhalten wird, wodurch die gezielte Entnahme des Buffy-Coats erschwert wird. Weiter ist erkannt worden, dass es wünschenswert ist, wenn die durch das Zentrifugieren erreichte Phasengrenze zwischen Erythrozyten und Buffy-Coat an einer vorbestimmten Position ausgebildet wird. Die Phasengrenze soll optimalerweise an der Grenze zwischen Fußabschnitt und Mittelabschnitt des Abtrennbehälters verlaufen, damit der Mittelabschnitt einzig für das Buffy-Coat, ggf. Blutplasma, jedenfalls nicht für die Erythrozyten, zur Verfügung steht und die Ausbeute nicht geschmälert wird. Schließlich ist erkannt worden, dass eine vorbestimmte Position der Phasengrenze erreicht werden kann, wenn anhand des Hämatokritwertes des zugeführten Blutes das künftige Packvolumen der zu zentrifugierenden Erythrozyten bestimmt wird, wenn der Fußabschnitt in seinem Aufnahmevolumen an das nach der Zentrifugierung zu erwartende Packvolumen der Erythrozyten derart angepasst wird, dass die sich beim Zentrifugieren ausbildende Phasengrenze zwischen Buffy-Coat und Erythrozyten in einem zum Fußabschnitt benachbarten Bereich des Mittelabschnittes des Abtrennbehälters positioniert wird, und wenn die Menge an zugeführtem Blut volumengenau, unter Berücksichtigung des zu erwartenden Packvolumens der Erythrozyten, in den Abtrennbehälter eingegeben wird. Bei dem Blut kann es sich bspw. um Nabelschnurblut oder Blut aus der Blutbank handeln, die jeweils mit Antikoagulans versetzt werden.

Zur Ermittlung des Hämatokritwertes könnten Standardwerte ausgewertet werden und daraus Rückschlüsse hinsichtlich des Packungsvolumens der Zellen der vorliegenden Blutprobe gezogen werden. In vorteilhafter Weise könnte eine viel genauere Bestimmung des Hämatokritwertes durch Probenahme aus dem zuzuführenden Blut ermittelt werden. Anhand des durch eine separate Zentrifugierung in einem einfachen Abtrennbehälter erhaltenen Hämatokritwertes kann dann das nach dem Zentrifugieren erwartete Packvolumen der Erythrozyten ermittelt werden und dann die Zuführung in den erfindungsgemäßen Abtrennbehälter erfolgen.

Aufgrund von Messungen kennt man das Verhältnis zwischen dem Packvolumen der Erythrozyten nach der Zentrifugierung. Zusammen mit dem ermittelten Hämatokritwert kann berechnet werden, welches Packvolumen an Erythrozyten entstehen wird, wenn zentrifugiert ist. Hat man beispielsweise eine Zuführmenge von 100 ml Nabelschnurblut, so ist abzusehen, dass zwischen 40 und 60 ml Packvolumen der Erythrozyten zu erwarten ist. Es muss also ein Aufnahmevolumen zwischen 40 und 60 ml am Fußabschnitt des Abtrennbehälters vorgesehen sein, das die Erythrozyten nach der Zentrifugierung aufnimmt.

Die Anpassung des Aufnahmevolumens des Fußabschnittes an das zu erwartende Packvolumen der Erythrozyten könnte durch Formgebungsänderungen realisiert werden. Dazu könnte der Fußabschnitt bspw. Kompartimente aufweisen, die abgeklemmt oder abgeschweißt oder abgebrochen werden. Auch eine Erweiterung des Fußabschnittes ist denkbar, wobei vorgefertigte Kompartimente über Brechventile verbunden werden können. Die Anpassung des Aufnahmevolumens könnte auch durch eine Auffüllmaßnahme erreicht werden. Bspw. könnte der Fußabschnitt mit inerten Kügelchen aufgefüllt werden, bis das verbleibende Aufnahmevolumen passend ist. Auf jeden Fall soll verhindert werden, dass Buffy-Coat in den Fußabschnitt oder in den Kopfabschnitt gelangt.

Im Fall des Abschweißens von Bereichen des Fußabschnitts findet eine irreversible Volumenänderung statt. Da immer auch ein experimenteller Faktor zu berücksichtigen ist, wie lange und wie schnell zentrifugiert wird, kann es zu Ungenauigkeiten kommen. Für den Fall, dass das Packvolumen etwas zu knapp berechnet wurde, würden Erythrozyten in den Mittelabschnitt gelangen. Für einen solchen Fall könnten kurzerhand Erythrozyten abgefühlt werden, so dass sich der Erythrozytenpegel senkt und der Mittelabschnitt wieder frei von Erythrozyten ist. Im Fall reversiblen Packvolumeneinstellung könnten - bspw. bei der Befüllung mit Kügelchen - diese im erforderlichen Maße entfernt werden oder auch Klemmen verschoben werden.

Die Anpassung des Aufnahmevolumens des Fußabschnittes an das nach der Zentrifugierung zu erwartenden Packvolumens der Erythrozyten könnte unter Zuhilfenahme einer horizontalen Skalierung am Fußabschnitt erfolgen. Es könnte auch mit einem Fußabschnitt gearbeitet werden, der verschiedene Kompartimente mit definierten Volumen aufweist und so eine Anpassung erzielt werden.

Ohne volumengenaue Eingabe des Blutes in den Abtrennbehälter könnte die optimale Aufnahme der Erythrozyten im Fußabschnitt nicht stattfinden. Die Zuführung des Blutes könnte deshalb vorzugsweise unter Zuhilfenahme einer vertikalen Skalierung am Abtrennbehälter erfolgen, die sich über alle drei Abschnitte (Kopfabschnitt, Mittelabschnitt, Fußabschnitt) erstreckt.

Zur Begünstigung der Sedimentation könnte dem Blut vor der Zentrifugierung eine weitere Substanz, insbesondere Hydroxyethylstärkelösung, im weiteren HES genannt, zugegeben werden. Die Zugabe könnte nach der Blutzugabe in den Abtrennbehälter erfolgen. Die erforderliche Menge an HES könnte unter Berücksichtigung der zugeführten Blutmenge berechnet werden und ebenfalls volumengenau zugegeben werden. Die HES-Zugabe könnte über die vertikale Skalierung kontrolliert werden.

Nach der Zentrifugierung erfolgt erfindungsgemäß die Abtrennung des Fußabschnittes vom Mittelabschnitt. Bevorzugt wird hier das sterile Abschweißen als Trennverfahren angewendet oder aber das Abklemmen. Beim Abtrennen des Fußabschnittes kommt es vor allem darauf an, dass die Abtrennung ohne Verluste für das Buffy-Coat erfolgt, d. h. geringste Mengen an Erythrozyten im Buffy-Coat können beim Abtrennvorgang eher in Kauf genommen werden. Selbstverständlich erfolgt auch eine Abtrennung des Mittelabschnitts vom Kopfabschnitt. Auf dieser Grundlage können mit dem hier stärker interessierenden Buffy-Coat zur Stammzellengewinnung weitere Verfahrensschritte durchgeführt werden.

Von wesentlicher Bedeutung für die Fortführung der vorliegenden Erfindung sind der Transport und die Lagerung des im Mittelabschnitt gesammelten Buffy-Coats. Nach einem bevorzugten Ausführungsbeispiel könnte das gesammelte Buffy-Coat in einen Einfrierbehälter entlassen werden, dessen Bestandteil der Mittelabschnitt selbst ist. Dazu wird die Verbindung zum zuvor, während der Zentrifugierung, abgetrennten Bereich des Einfrierbehälters hergestellt. Bei diesem bevorzugten Ausführungsbeispiel wird Bauhöhe gespart, da der erfindungsgemäße Abtrennbehälter in einen standardisierten Zentrifugenbecher passen muss.

Nach einem alternativen Ausführungsbeispiel könnte das Buffy-Coat aber auch aus dem Mittelabschnitt in einen Einfrierbehälter gelangen, der vorzugsweise am Kopfabschnitt des Abtrennbehälters angeordnet und mit diesem bspw. über eine steril konnektierte Leitung strömungsverbindbar ist. Nach der Zentrifugierung und nach Abtrennung des Fußabschnittes vom Mittelabschnitt könnte dann die Strömungsverbindung zwischen Einfrierbehälter und Mittelabschnitt hergestellt werden. Bei diesem Ausführungsbeispiel ist es von Vorteil, zwei Zentrifugierungen vorzunehmen, wobei die zweite Zentrifugierung nach Abtrennung des Fußabschnittes in entgegengesetzter Richtung stattfindet und dazu dient, das Buffy-Coat in den Kopfabschnitt und schließlich in den daran angeschlossenen, strömungsverbundenen Einfrierbehälter und das leichtere Blutplasma in den Mittelabschnitt zu befördern. Der bspw. durch eine Schweißnaht abgetrennte, aber noch vorhandene Fußabschnitt könnte den zweiten Zentrifugiervorgang mit durchlaufen. Der abgetrennte Fußabschnitt könnte umgeschlagen werden, da bei der zweiten Zentrifugierung der Einfrierbehälter direkt befüllt wird und die Bauhöhe des standardisierten Zentrifugenbechers berücksichtigt werden muss. Die Verbindungsleitung zwischen Einfrierbehälter und Abtrennbehälter könnte in einem Stabilisator gehalten sein.

Zur Zentrifugierungsbeschleunigung ist zu bemerken, dass bei Blut, das mit HES versetzt wurde und wobei ein zweiter Zentriervorgang geplant ist, ein geringer Wert ausreichend ist. Bei der zweiten Zentrifugierung muss dann erheblich beschleunigt werden, um das Buffy-Coat hinreichend im dafür vorgesehenen Abschnitt einzuengen.

Die Vorgänge betreffend das zentrifugierte Buffy-Coat im Einfrierbehälter könnten gemäß der erfindungsgemäßen Weiterbildung des Verfahrens dahingehend ausgestaltet werden, dass auch der Einfrierbehälter - wie zuvor der Fußabschnitt - hinsichtlich seines Aufnahmevolumens an die nach der Zentrifugierung sichtbaren Menge an Buffy-Coat angepasst wird. Die Anpassung könnte bspw. durch die Abtrennung von Kompartimenten des Ehfrierbehälters erfolgen. Die Abtrennung der strömungsverbundenen Kompartimente könnte durch eingearbeitete Stege innerhalb des Einfrierbehälters erleichtert werden. Es ist klar, dass der Einfrierbehälter nach der Einstellung des Aufnahmevolumens und nach der Befüllung mit dem Buffy-Coat oder ggf. mindestens einer weiteren Substanz komplett abgedichtet und schließlich auch vom Kopfabschnitt abgetrennt werden muss. Auf diese Weise werden kleine, handliche Einfrierbehälter geschaffen, die optimal befüllt sind und keinen überflüssigen Platz in den teuren Blutbanken / Tieftemperatureinrichtungen wegnehmen. Bei der Einstellung des Aufnahmevolumens des Einfrierbehälters zur Buffy-Coataufnahme ist zu berücksichtigen, dass weitere Substanzen, insbesondere DMSO, ggf. Blutplasma oder eine Mischung daraus, bei der Einstellung des Aufnahmevolumens zu berücksichtigen sind.

Alternativ zu einer Substanzzufuhr über eine Zuführleitung, könnte im Einfrierbehälter bereits vorab ein Reservoir mit DMSO bereitgestellt werden. Beispielsweise könnte ein Bereich am Einfrierbehälter abgeklemmt werden, um das Reservoir zu erzeugen. Wenn der Einfrierbehälter über Kompartimente verfügt, könnte das Reservoir auch durch mindestens ein Kompartiment gebildet werden. Nachdem die Befüllung des Einfrierbehälters mit Buffy-Coat stattgefunden hat, könnte die Verbindung zwischen den einerseits mit DMSO, andererseits mit Buffy-Coat gefüllten Kompartimenten des Einfrierbehälters hergestellt werden und alles könnte im Einfrierbehälter vermischt werden.

Wenn die Menge an Buffy-Coat für ein das Standardvolumen des Einfrierbehälters nicht ausreichend ist, könnte Blutplasma als Füllstoff zugegeben werden. Das Blutplasma könnte ebenfalls in ein Reservoir des Einfrierbehälters eingegeben werden. Alternativ könnte in das Reservoir eine Mischung aus UMSO und Blutplasma eingeführt werden.

Bei der Zentrifugierung von Blutproben und sämtlichen Folgeschritten ist eine Verunreinigung unerwünscht. Das Kontaminationsrisiko könnte sehr stark vermindert werden, wenn die Befüllung des bekanntermaßen nach außen dichten Abtrennbehälters der Zentrifuge und die Entnahme daraus unter Ausschluss der Atmosphäre stattfindet. Der Kontakt mit der Atmosphäre könnte ausgeschlossen werden, wenn die Befüllung des Abtrennbehälters mit Blut über dessen steril konnektierbaren Zuführanschluss erfolgt. Auch die Zugabe weiterer Substanzen in die Zuführeinrichtung für Blut, in den Abtrennbehälters oder in den Einfrierbehälter sollte über sterile Verbindungen erfolgen. Dabei könnten der Abtrennbehälter, der Einfrierbehälter und alle weiteren Entnahme- und / oder Zuführeinrichtungen mit Vorrats- und/oder Misch- und/oder Dosier- und/oder Transportfunktion miteinander steril konnektiert sein.

Die Zuführeinrichtung für Blut könnte einen Blutsammelbehälter umfassen, in den das über Kanülen entnommene Blut eingespeist wird. Auch der Blutsammelbehälter könnte einen sterilen Anschluss aufweisen und gegen die Umwelt abgedichtet sein. Auf diese Weise wird auch innerhalb der Zuführeinrichtung, die aufgrund der zur Blutentnahme offenen Kanülen an sich kein geschlossenes System ausbilden kann, dennoch nach Eintritt in den Blutsammelbehälter eine Komponente erzeugt, die verschließbar ist und; auch Bestandteil eines geschlossenen Systems sein kann. Der Blutsammelbehälter könnte in Form eines kollabierten Beutels vorliegen und daher auch ohne Verdrängung von Luft mit Blut befüllbar sein.

Im Gegensatz zu einem kollabierten Beutel, der unbefüllt zweidimensional ist und erst nach der Befüllung eine Dreidimensionalität erlangt, könnten die für das Blut und seine Fraktionen vorgesehenen Abschnitte des Abtrennbehälters von Beginn an dreidimensional sein und so in vorteilhafter Weise eine viel größere Aufnahmekapazität für das Blut bereitstellen. Der Nachteil der kollabierten Beutel liegt in der Limitierung der Blut- und ggf. Hilfsstoffvolumina, verbunden mit der Notwendigkeit mehrerer Zentrifugierarbeitsgänge, Teilung der aus einer Quelle stammenden Blutmenge sowie deren Kennzeichnung und Verwaltung. Wird ein kollabierter Beutel noch in Kammern unterteilt, reduziert sich das Aufnahmevermögen noch mehr. Die kollabierten Beutel weisen standardisierte Maße auf, so dass wenig Spielraum besteht. Es ist für die Erfindung wesentlich, dass das Blut in dreidimensionale Abtrennbehälterabschnitte gelangt, da so variable Einfüllvolumina realisiert werden können. Durch die vorteilhafte Dreidimensionalität der Abtrennbehälterabschnitte von Beginn an können variäbel auch größere Mengen an Blut, ggf. Hilfsstoffen in einem Arbeitsgang zentrifugiert werden. Es können Einfüllvolumina bis zu ca. 500 ml realisiert werden.

In vorteilhafter Weise könnten alle Komponenten jeweils ein geschlossenes System ausbilden, die untereinander steril in Verbindung gebracht werden oder deren Verbindung unterbrochen werden können oder die völlig voneinander steril abgetrennt werden. Aus den einzelnen geschlossenen Systemen wird nach steriler Konnektierung ein geschlossenes Gesamtsystem, das schließlich wieder in einzelne geschlossene Systeme zerlegt werden kann. Es ist offensichtlich, dass das Kontaminationsrisiko in vorteilhafter Weise auf den Schritt der Blutentnahme reduziert wird.

Gerade die DMSO-Zugabe findet in der Praxis stets unter Öffnung des Behälters statt, der das Buffy-Coat enthält. Hierbei kann es zur Kontamination kommen. Man begegnet dieser Gefahr durch das Arbeiten in teuren Reinräumen. Das erfindungsgemäße Ausführungsbeispiel, das auch durch das erfindungsgemäße System reflektiert wird, ermöglicht in vorteilhafter Weise eine Kostenreduktion, da man auch im Labor arbeiten kann, wenn alle Komponenten, die der Zuführeinrichtung oder der Blutzuführleitung zu einem Blutsammelbehälter der Zuführeinrichtung nachgeordnet sind, Bestandteile eines geschlossenen Systems sind. Dabei könnte es sich beim Blutsammelbehälter um einen kollabierten Beutel handeln, der die Dreidimensionalität erst durch Einspeisen des Blutes erlangt. Die Leitungen sind mit öffenund verschließbaren Anschlüssen am Blutsammelbehälter steril verschweißt, so dass das geschlossene System durch Schließung der Blutzuleitung hergestellt werden kann.

Bei dem bevorzugten Ausführungsbeispiel, wobei die Bluteinspeisung in den von Beginn an dreidimensionalen Abtrennbehälter erfolgt, startet das geschlossene System mit der Verschließung des Abtrennbehälters nach Befüllung. Die im Abtrennbehälter vorhandene Luft kann in einen zusätzlichen Behälter verdrängt werden oder - im Fall das ein Blutsammelbehälter vorgesehen ist - in diesen geleitet werden. Im letzteren Fall findet eine systeminterne Zirkulation von Luft / Inertgas vom Abtrennbehälter zum Blutsammelbehälter und von Blut vom Blutsammelbehälter zum Abtrennbehälter statt.

Was nun die DMSO-Zugabe angeht, so sind Spritze und DMSO-Ampulle innerhalb von dreidimensionalen Behältern enthalten oder sind so ummantelt, das der Benutzer dennoch-durch die trennende Wandung hindurch - die Spritze betätigen kann und die Ampulle öffnen und platzieren kann. Die Leitungen sind ebenfalls steril an die Behälter / Ummantelung angeschlossen, so dass kein Kontakt mit der Außenwelt stattfindet. Auch die weiter zuzugebende Substanz HES ist in einem Behälter enthalten, der einen Raum aufspannt, in dem die Substanz enthalten ist und der steril angeschlossene Leitungen aufweist. Es findet somit keine Öffnung des Systems statt. Vielmehr werden die Substanzen ohne Austausch mit der Umwelt transportiert, die Luft oder das Inertgas verbleibt ebenfalls im System, das Vorkehrungen zum Druckausgleich umfasst. Von Luft- oder Gasabführung über Filter wird ausdrücklich abgesehen, da stets die Gefahr des Eintritts von Gas und Viren in das System besteht. Die verdrängte Luft wird nur dann vom System entfernt, wenn eine sterile Abtrennung von Komponenten gemäß den aufeinanderfolgenden Verfahrensschritten erfolgt.

Auch außerhalb des geschlossenen Systems, im Bereich der Zuführeinrichtung, wird Antikoagulans in einem Behälter gelagert, der für die Aufnahme des Antikoagulans einen Raum aufspannt und steril angeschlossene Leitungen aufweist.

Sobald der Abtrennbehälter mit Blut befüllt ist, wird die Zuführeinrichtung für Blut steril diskonnektiert. Dagegen werden die Entnahme- und / oder Zuführeinrichtungen mit Vorrats- und/oder Misch- und/oder Transport- und/oder Dosierfunktion betreffend DMSO, auch mit der gesamten Spritzenmontur, Blutplasma, HES, ggf. inerte Kügelchen mit dem Abtrenribehälter nebst allen Verbindungsleitungen gemeinsam in einen standardisierten Zentrifugenbecher eingebracht. Die Verbindungsleitungen sind mit den Komponenten steril verbundenen, aber durch Schlauchklemmen gesichert, so dass die Strömungsverbindung erst durch Entfernen der Schlauchklemmen hergestellt werden kann. Für den Fall, dass der Abtrennbehälter Bestandteil eines geschlossenen Systems ist, könnte auch eine Druckausgleicheinrichtung am Abtrennbehälter oder an der Ummantelung einer Spritze, die DMSO zuführt, in den Zentrifugenbecher eingebracht werden. Dies gilt auch für einen Aufnahmebehälter für Erythrozyten, der am Fußabschnitt des Abtrennbehälters angeordnet sein kann und nach dem Zentrifugieren überschüssige Erythrozyten aufnimmt, falls die Phasengrenze bzw. der Buffy-Coat-Spiegel zu hoch im Mittelteil steht und durch den Zentrifugiervorgang eine Abweichung von der Berechnung eingetreten sein sollte.

Spätestens nachdem der Einfrierbehälter mit Buffy-Coat und gg. DMSO, ggf. Blutplasma befüllt ist, werden übrige Bestandteile des Abtrennbehälters steril vom Einfrierbehälter entfernt / abgeschweißt. Der Einfrierbehälter kann nun der Einfrierprozedur in der Blutbank zugeführt werden. Bei einem Einfrierbehälter mit Kompartimenten können Teile vom Einfrierbehälter verschiedenen Anwendungen und Lagerorten zugeführt werden. Nach einem weiteren Ausführungsbeispiel könnte die Abtrennung des Einfrierbehälters vom Abtrennbehälter oder von Resten des Abtrennbehälters auch vor der Befüllung mit DMSO erfolgen.

Im Hinblick auf den erfindungsgemäßen Abtrennbehälter ist erkannt worden, dass durch die Anpassbarkeit des Aufnahmevolumens des Fußabschnittes an das nach der Zentrifugierung zu erwartende Packvolumen der Erythrozyten die Phasengrenze genau, nämlich in einem Bereich nahe der Grenze zwischen Fuß- und Mittelabschnitt, positioniert werden kann und damit die Buffy-Coat-Ausbeute im Mittelabschnitt optimiert werden kann.

Damit eine volumengenaue Zugabe der Blutprobe erfolgen kann, könnte am Abtrennbehälter eine über alle Abschnitte reichende vertikale Skalierung vorgesehen sein. Zur Voreinstellung des Aufnahmevolumens des Fußabschnittes entsprechend dem nach der Zentrifugierung zu erwartenden Packvolumens der Erythrozyten könnte am Fußabschnitt eine horizontale Skalierung vorgesehen sein.

Um nun das Aufnahmevolumen des Fußabschnittes an das zu erwartende Packvolumen anzugleichen, insbesondere zu verkleinern, könnten Klemmvorrichtungen am Fußabschnitt anbringbar sein. Nach einer weiteren Ausführungsform könnte der Fußabschnitt strömungsverbindbare Kompartimente aufweisen, die zur Verkleinerung des Volumens abschweißbar sind. Bei diesem Ausführungsbeispiel, wobei der Fußabschnitt aus Kompartimenten aufgebaut ist, könnten die Kompartimente bei Bedarf über Abbrechventile strömungsverbindbar sein. Durch diese Ausführungsform kann auch eine Vergrößerung des Volumens herbeigeführt werden. Es könnten auch Bereiche des Fußabschnittes einfach abschweißbar sein.

Insbesondere im Fall der Irreversibilität der Volumenverkleinerung, aber auch generell, könnte am Fußabschnitt ein Aufnahmebehälter zum Abführen von zentrifugierten Erythrozyten vorgesehen sein, um den Mittelabschnitt frei von Erythrozyten zu halten. Der Aufnahmebehälter für Erythrozyten könnte insbesondere steril mit dem fußabschnittseitigen Anschluss des Abtrennbehälters verbunden sein. Sollte also nach der Zentrifugierung die Phasengrenze zu weit oben im Mittelteil platziert sein, können kurzerhand Erythrozyten aus dem Fußabschnitt in den Aufnahmebehälter abgelassen werden. Nach dieser ggf. erforderlichen Aufnahme von Erythrozyten könnte der Aufnahmebehälter vorzugsweise steril vom Abtrennbehälter abtrennbar sein

Schließlich könnte alternativ oder zusätzlich eine Zuführeinrichtung für Füllstoff, insbesondere inerte Kügelchen vorgesehen sein, die mit dem Fußabschnitt strömungsverbindbar ist, so dass zur Verkleinerung des Volumens des Fußabschnittes Füllstoff in diesen einbringbar ist. Damit die als für die Erfindung sehr vorteilhaft erkannte Dreidimensionalität der für die Blutfraktionen vorgesehenen Abschnitte des Abtrennbehälters erhalten werden, könnte der Abtrennbehälter in fertigungstechnischer Hinsicht aus zwei übereinanderliegenden Folien mit tiefgezogenen Bereichen für die Ausbildung der verschiedenen Abschnitte und einem dazwischenliegenden Blindbereich gefertigt sein. Der Blindbereich außerhalb der Abschnitte für die Blutfraktionen könnte flach und verschweißt sein. Auf diese Weise entstehen einerseits in den tiefgezogenen Bereichen die verschiedenen Abschnitte zum Aufnehmen der verschiedenen Blutfraktionen - Fußabschnitt, Mittelabschnitt, Kopfabschnitt - und andererseits Blindbereiche, die kaum Platz beanspruchen, sondern ermöglichen, dass Zuführeinrichtungen für DMSO, HES oder Füllstoff nebst Verbindungsleitungen mit im Zentrifugenbecher untergebracht werden können. Am Rand des Blindbereiches könnten Vorkehrungen getroffen sein, damit der Abtrennbehälter in einem Einsatz befestigt werden kann, der dann in den Zentrifugenbecher eingelassen wird. Der erfindungsgemäße Abtrennbehälter ist in seiner von Beginn an vorhandenen Dreidimensionalität - mit oder ohne Blindbereiche - für eine normale Blutbankzentrifuge geeignet und passt in standardisierte Zentrifugenbecher, auch wenn weitere Bestandteile des Systems zusätzliche auch noch im Zentrifugenbecher unterzubringen sind.

Im Blindbereich könnten diverse schlitzförmige Öffnungen vorgesehen sein, durch die Klemmvorrichtungen, wie bspw. eine Klemmzange, hindurchgeführt werden können. Dies ist zum einen im Bereich des Mittelabschnittes erforderlich, damit zumindest unterhalb der Phasengrenze abgeklemmt und das Buffy-Coat gesichert werden kann, aber auch im Bereich des Fußabschnittes und - bei einer bevorzugten Ausgestaltung des erfindungsgemäßen Abtrennbehälters auch im Zusammenhang mit der Transformation des Mittelabschnitts zum Einfrierbehälter. Durch die schlitzförmigen Öffnungen könnte auch eine Schweißvorrichtung zum Abtrennen der Abschnitte voneinander oder zum Abtrennen von Bereichen eines Abschnitts, insbesondere des Fußabschnitts bei der Volumenverkleinerung, hindurchführbar sein.

Im Zentrifugenbecher können Aufhängungen vorgesehen sein, in denen Befestigungsmittel des Abtrennbehälters verankert werden. Hier kommt vor allem der dem Kopfabschnitt benachbarte und nach oben abschließende Rand des Blindbereiches in Betracht, der eine Reihe von Durchtrittsöffnungen aufweisen könnte. Diese können auch bei Aufhängungen an Stativen während der Durchführung anderer Verfahrensschritte nützlich sein. Zusätzlich könnte ein Stabilisierungsteil bzw. ein Einsatz in den Zentrifugenbecher eingebracht werden, das bzw. der den erfindungsgemäßen Abtrennbehälter stützt und auf dessen Formgebung abgestimmt ist.

Eine vorteilhafte Weiterbildung des Abtrennbehälters im Hinblick auf das Ziel, Buffy-Coat der Kryokonservierung zuzuführen, sieht vor, dass dieser einen Einfrierbehälter umfasst. Aus Sicherheitsgründen im Sinne einer Redundanzmaßnahme könnten einem Abtrennbehälter auch zwei Einfrierbehälter zugeordnet sein können, die beide befüllt werden. Dies ist im Hinblick auf die Hinterlegung des Buffy-Coats an zwei verschiedenen Orten vorteilhaft. Möglich ist aber auch ein Einfrierbehälter mit Kompartimenten, die im Nachhinein abtrennbar sind.

Nach einer ersten, bevorzugten Alternative könnte der Einfrierbehälter integraler Bestandteil des Mittelabschnitts des Abtrennbehälters sein. Dies ist eine platzsparende Variante, die sehr gut auch in einem standardisierten Zentrifugenbecher Platz findet. Durch das Abschweißen von Kopf- und Fußabschnitt nach der Zentrifugierung und nach Einlass des Buffy-Coats und ggf. weiterer Substanzen in den Einfrierbehälter steht dann ein Einfrierbehälter zur Verfügung, in den dann - andersherum betrachtet - der Mittelabschnitt des einstigen Abtrennbehälters integriert ist. Der Einfrierbehälter könnte über eine Perforationslinie aus dem oben beschriebenen Blindfeld herausgetrennt werden.

Alternativ könnte der Einfrierbehälter dem Abtrennbehälter auch zugeordnet und über öffenund verschließbare Leitungen mit diesem strömungsverbindbar sein. Hier könnte die Verbindung am Kopfabschnitt stattfinden, wobei dann ein zweiter Zentrifugierungsvorgang in entgegengesetzter Richtung erforderlich ist, der bereits in Bezug auf das Verfahren beschrieben ist.

Insbesondere wenn□das Ziel verfolgt wird, gemäß einer bevorzugten Ausführung im geschlossenen System - ohne Austausch mit der Umwelt, also ohne Filter - zu arbeiten, wird dem Abtrennbehälter eine Druckausgleicheinrichtung für Luft oder Inertgas zugeordnet, die steril angeschlossen und auch steril abtrennbar ist. Bezogen auf das Ausführungsbeispiel, wobei der Mittelabschnitt als Einfrierbehälter umgebildet wird, könnte die Druckausgleicheinrichtung bspw. am Mittelabschnitt des Abtrennbehälters steril angeschlossen sein. Bei einem Ausführungsbeispiel mit Blutsammelbehälter könne auch dieser zum Druckausgleich bzw. zum Verdrängen von Luft / Inertgas verwendet werden. Eine Druckausgleicheinrichtung ist auch dann erforderlich, wenn der Abtrennbehälter von Beginn ah dreidimensionale Bereiche zur Ausbildung von Kopf,- Mittel- und Fußabschnitt aufweist. Lufteinschlüsse spielen bei dreidimensionalen Behältern stets eine Rolle. Lufteinschlüsse können auf das Einfriergut und den Einfrierbehälter eine negative Auswirkung haben. Gelangt Luft in den Einfrierbehälter, so kann es zu Spannungen kommen und gerade bei den niedrigen Einfriertemperatren von nahezu - 200 C° kann der Einfrierbehälter reißen.

Das Aufnahmevolumen des Einfrierbehälters könnte an die zu erwartende Ausbeute an zentrifugiertem Buffy-Coat aus dem Mittelabschnitt, ggf. an die Zugabe mindestens einer weiteren Substanz, anpassbar sein. Hierzu könnte der Einfrierbehälter strömungsverbindbare Kompartimente, insbesondere mit zwischen den Kompartimenten eingearbeiteten Stegen, aufweisen, die zur Volumenverkleinerung abtrennbar, insbesondere steril abschweißbar, sind.

Wie bereits im Zusammenhang mit dem Verfahren beschrieben, könnte das Blut einer Nabelschnur entnommen werden oder es könnte sich um antikoaguliertes Blut einer Blutbank handeln. Die Zugabe von Blut könnte am Fußabschnitt des Abtrennbehälters über einen steril konnektierbaren Anschluss erfolgen. Bei Nabelschnurblut könnte die Zuführeinrichtung zumindest eine Kanüle zur Blutnahme, eine daran angeordnete Verbindungsleitung und zumindest eine Abzweigung zum Anschluss einer Zuführeinrichtung für Antikoagulans an die Verbindungsleitung aufweisen. Außerdem könnte noch am Anschluss an den Abtrennbehälter ein Probenahmeabschnitt als tote Abzweigung der Verbindungsleitung vorgesehen sein. Die aus dem Probenahmeabschnitt entnommene Blutprobe kann zur Hämatokritwertbestimmung im Vorfeld benutzt werden, um das nach der Zentrifugierung zu erwartende Packvolumen der Erythrozyten patientenspezifisch zu bestimmen. Der Probenahmeabschnitt könnte verschiedene Abmessungen, insbesondere Längenabmessungen, aufweisen. Auf diese Weise könnten Proben zusätzlich zur Hämatokritwertbestimmung zur Verfügung gestellt werden.

Damit das Kontaminationsrisiko sehr klein gehalten wird, könnte die Kanüle eine perforierbare sterilisierte Latexumhüllung aufweisen, die erst durchstochen wird, wenn die Nabelschnur mit der Kanüle punktiert wird. Das Zeitfenster eines Umweltkontakts wird dadurch sehr klein gehalten und die Kontaminationsgefahr wird verringert.

Über eine Abzweigung könnte an die Verbindungsleitung der Zuführeinrichtung für Blut eine Zuführeinrichtung für HES zur Unterstützung der Sedimentation angekoppelt sein. Alternativ könnte die Zuführeinrichtung auch einen Blutsammelbehälter umfassen und die Zuführeinrichtung für HES könnte vorzugsweise steril mit dem Blutsammelbehälter verbunden sein. Nach einer weiteren Ausführungsform könnte die HES-Zufuhr auch direkt über einen Anschluss an den Abtrennbehälter erfolgen. Die Substanz könnte in einem Vorratsbehälter mit Anschluss enthalten sein oder auch in einer Ampulle, die erst bei Bedarf geöffnet wird. Im letztgenannten Fall würde bei Wunsch nach Arbeiten im geschlossenen System ein Behälter vorgesehen sein, der die Ampulle umhüllt - analog zur DMSO-Zuführeinrichtung Auch hier ist dafür zu sorgen, dass verdrängte Luft im geschlossenen System verbleibt - bspw. über eine steril angeschlossene Druckausgleicheinrichtung. Auf HES könnte dann völlig verzichtet werden, wenn die Zentrifugierung bei höheren g-Werten (Beschleunigungswerten) bzw. bei höheren Umdrehungsgeschwindigkeiten stattfindet.

Auch der Einfrierbehälter - ob separat oder am Mittelabschnitt des Abtrennbehälters angeordnet - und ggf. der Kopfabschnitt des Abtrennbehälters könnten mit weiteren Entnahmeund / oder Zuführeinrichtungen mit Transport-, Vorrats- und/oder Misch- und/oder Dosierfunktion verbunden sein. Aus bereits dargestellten Gründen könnte der Einfrierbehälter mit einer Zuführeinrichtung für DMSO verbunden sein. Dort könnte das DMSO über eine Abbrechampulle zur Verfügung stehen. Nach Zerbrechen könnte das DMSO über einen Partikelfilter / Rundfilter manuell über die Spritze entnommen und dann über eine Spritzenpumpe zum Einfrierbehälter gepumpt werden. Die Befüllung mit DMSO erfolgt erst nach der Zentrifugierung. Der Kopfabschnitt des Abtrennbehälters könnte mit einer Entnahmeeinrichtung zur Entnahme von Blutplasma verbunden sein. Zum Erreichen eines standardisierten Volumens im Einfrierbehälter im Rahmen der Kryokonservierung kann auch Blutplasma aus dem Kopfabschnitt - bspw. über die Spritze - entnommen und zum Einfrierbehälter geleitet werden.

Der Abtrennbehälter, der Einfrierbehälter, die Zuführeinrichtung für DMSO und die Spritze, die Zuführeinrichtung für HES - unabhängig davon, ob am Abtrennbehälter, an einem Blutsammelbehälter oder an der Zuführleitung zum Abtrennbehälter angeschlossen - , ggf. der Blutsammelbehälter der Zuführeinrichtung für Blut, könnten Bestandteile eines geschlossenen Systems sein. Dabei könnten die Einzelkomponenten innerhalb von Behältern enthalten sein, die im Sinne von sterilen Ummantelungen ausgebildet sind und keinen Austausch mit der Umwelt zulassen. Zur Ausbildung des geschlossenen Systems könnten die Einzelkomponenten steril konnektiert sein. Ein System ermöglicht für alle Zuführ- und Entnahmeeinrichtungen eine gemeinsame Transport- und Mischlösung. Dies betrifft insbesondere die Spritze, die zum Transport von DMSO, ggf. Blutplasma, ggf. HES vorgesehen ist und auch als Mischbehältnis, insbesondere für Blutplasma und DMSO, dient.

Zur Reduzierung des Systems je nach Verfahrensfortschritt könnten die Einzelkomponenten voneinander steril abtrennbar sein. Zur reversiblen Unterbrechung der Strömungsverbindung könnten Schlauchklemmen vorgesehen sein. Der Gedanke des geschlossenen Systems-unter Ausnehmung der Blutentnahme als einzigen "offenen" Punkt - in der vorbeschriebenen Ausführungsform ist für die Erfindung von großer Bedeutung, da die Kontamination dann nahezu ausgeschlossen ist. Nur noch bei der Blutentnahme kann Kontamination eintreten. Nach Eintritt in den Abtrennbehälter nach einer Ausgestaltung oder in den Blutsammelbehälter nach einer anderen Ausgestaltung kann keine zusätzliche Kontamination mehr erfolgen.

Eine erfindungswesentliche Weiterbildung des erfindungsgemäßen Abtrennbehälters besteht darin, dass am Fuß-, Mittel- und Kopfabschnitt des Abtrennbehälters, ggf. an einem Probenahmeabschnitt, mindestens eine Identifikationsnummer aufgebracht ist. Der Probenahmeabschnitt kann ein toter Abzweig von einer Verbindungsleitung sein, die zum Abtrennbehälter führt. Der Probenahmeabschnitt könnte nach einer Ausführungsform auch an einer Verbindungsleitung abzweigen, die mit dem Blutsammelbehälter verbunden ist und zum Abtrennbehälter führt Diese Identifikationsnummern vermeiden in vorteilhafter Weise die aufwendige nachträgliche Identifizierung. Von Beginn an besteht der Zusammenhang zwischen Blutentnahme und damit dem Patienten und der korrekt patientenbezogenen Hämatokritwertbestimmung und schließlich dem Einfrierbehälter, der dieselben Identifikationsnummern aufweist und somit die Gefahr von falschen Zuordnungen ausgeschlossen ist.

Die Beschreibung des Verfahrens und seiner Ausgestaltungen trifft auch auf den Abtrennbehälter zu, so dass zur Erläuterung des Abtrennbehälters auf die dortigen Ausführungen Bezug genommen wird.

Im Hinblick auf das erfindungsgemäße System ist erkannt worden, dass der erfindungsgemäße Abtrennbehälter, der einen Einfrierbehälter umfasst und das erfindungsgemäße Verfahren, das die Befüllung des Einfrierbehälters mit Buffy-Coat umfasst, innerhalb eines Systems angewendet werden können, das ab dem Schritt der Blutentnahme Kontaminationsfreiheit ermöglicht. Die einzige Kontaminierung kann also nur am Beginn des Verfahrens stattfinden, danach nicht mehr. Zudem kann durch das System ein Mittel an die Hand gegeben werden, dessen Bestandteile nach sukzessiver Ausführung der verschiedenen Verfahrensschritte nach und nach reduziert werden, bis der Einfrierbehälter mit dem präparierten Buffy-Coat übrig bleibt. Das System steht in direktem technologischen Zusammenhang mit dem erfindungsgemäßen Verfahren und dem erfindungsgemäßen Abtrennbehälter gemäß bestimmten Ausführungsformen. Darüber hinaus kann es aber auch grundsätzlich ohne den Schritt der vorherigen Hämatokritwertbestimmung und Volumenmanipulation am Fußabschnitt des Abtrennbehälters verwendet werden, insbesondere dann, wenn ein Aufnahmebehälter für Erythrozyten vorgesehen ist.

Besonders bevorzugt wird ein System, dessen Blutzuführung einen Blutsammelbehälter umfasst. Hier kann zunächst das Antikoagulans eingefüllt werden, dass sich mit dem später zugeführten abgenommenen Blut vermischt. Zudem kann in einem nachgeschalteten Schritt die Blutzuführleitung zum Blutsammelbehälter mit Antikoagulans durchspült werden und so der letzte Tropfen Blut zum Blutsammelbehälter transportiert werden. Das abgenommene Blut wird also weitgehend verlustfrei gesammelt. Wenn die Blutsammlung abgeschlossen ist, kann die Zuführleitung steril abgeschweißt werden und das um die Kanülen und die Antikoagulansbehälter reduzierte System wird zur Blutbank transportiert. Dort wird das System an einem Stativ aufgehängt. Auch dort erweist sich der Blutsammelbehälter als vorteilhaft, da nun sehr bequem Blut zunächst in einen Probenahmeabschnitt später in den Abtrennbehälter eingefüllt werden kann. Im Hinblick auf die Ausbildung eines geschlossenen Systems nach Schließung des Blutsammelbehälters und die ggf. erforderliche HES-Zugabe wird auf die Beschreibung zum Verfahren und zum Abtrennbehälter verwiesen.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche, andererseits auf die nachfolgende Erläuterung von fünf Ausführungsbeispielen des erfindungsgemäßen Abtrennbehälters und zwei Aüsführungsbeispielen des erfindungsgemäßen Systems anhand der Zeichnung zu verweisen. In Verbindung mit der Erläuterung der angeführten Ausführungsbeispiele der Erfindung werden auch im Allgemeinen bevorzugte Ausgestaltungen und Weiterbildungen der Lehre erläutert. In der Zeichnung zeigen
- Fig. 1: in schematischer Darstellung, den erfindungsgemäßen Abtrennbehälter gemäß einem ersten Ausführungsbeispiel, wobei der Einfrierbehälter dem Mittelabschnitt zugeordnet ist,
- Fig. 2: in schematischer Darstellung eine Ansicht eines ersten Beispiels für ein erfindungsgemäßen Systems mit einer Zuführungeinrichtung für Blut, einem Abtrennbehälter gemäß einem zweiten Ausführungsbeispiel, wobei der Einfrierbehälter an den Abtrennbehälter angeschlossen ist und weiteren Zugabemöglichkeiten für Blutplasma und HES einerseits sowie Blutplasma und DMSO andererseits zum Zeitpunkt vor der Blutentnahme aus der Nabelschnur,
- Fig. 3: in schematischer Darstellung einen erfindungsgemäßen Abtrennbehälter gemäß einem dritten Ausführungsbeispiel, wobei der Einfrierbehälter an den Abtrennbehälter angeschlossen ist und Zugabemöglichkeiten im Hinblick auf DMSO und HES bestehen,
- Fig. 4: in schematischer Darstellung einen erfindungsgemäßen Abtrennbehälter gemäß einem vierten Ausführungsbeispiel, wobei der Einfrierbehälter an den Abtrennbehälter angeschlossen ist und eine Füllstoffzuführung zur Verkleinerung des Aufnahmevolumens des Fußabschnitts vorgesehen ist,
- Fig. 5: in schematischer Darstellung eine Ansicht eines zweiten Beispiels des erfindungsgemäßen Systems mit Zuführungeinrichtung für Blut, Abtrennbehälter gemäß einem fünften Ausführungsbeispiel und weiteren Zugabeeinrichtungen, wobei der Einfrierbehälter durch den Mittelabschnitt des Abtrennbehälters ausbildbar ist - zum Zeitpunkt kurz nach der Blutentnahme aus der Nabelschnur,
- Fig. 6: in schematischer Darstellung eine Ansicht des Systems gemäß Fig. 5, wobei Kanülen, Antikoagulans-Zuführeinrichtungen und Probenahmeabschnitt bereits steril abgetrennt sind und begonnen wird, den Abtrennbehälter mit Blut aus dem Blutsammelbehälter zu befüllen,
- Fig. 7: in schematischer Darstellung eine Ansicht des Systems gemäß Fig. 6, wobei HES- und Blutsammelbehälter bereits steril abgetrennt sind und das System in einen Zentrifugenbecher eingebracht wird,
- Fig. 8: in schematischer Darstellung eine Ansicht des Systems gemäß Fig. 7, nach dem Zentrifugieren,
- Fig. 9: in schematischer Darstellung eine Ansicht des Systems gemäß Fig. 8, wobei der Fußabschnitt steril abgetrennt ist und Buffy-Coat vom Mittelabschnitt in ein weiteres Kompartiment des Einfrierbehälters eingeflößt wird,
- Fig. 10: in schematischer Darstellung eine Draufsicht auf das System aus Fig. 9, jedoch mit abgeschweißter DMSO-Zuführeinrichtung, betreffend die DMSO Zugabe über die Spritze,
- Fig. 11: in schematischer Darstellung eine Draufsicht auf das System aus Fig. 10, jedoch mit steril abgeschweißter DMSO-Leitung, betreffend die Verteilung der Mischung aus Buffy-Coat, Blutplasma und DMSO in allen Kompartimenten des Einfrierbehälters mittels Matrize und
- Fig. 12: in schematischer Darstellung eine Draufsicht auf das System aus Fig. 10, jedoch mit steril abgeschweißter Luftleitung und steril getrennter Verbindungsleitung sowie der Option auf Teilung des Einfrierbehälters in zwei Teile.

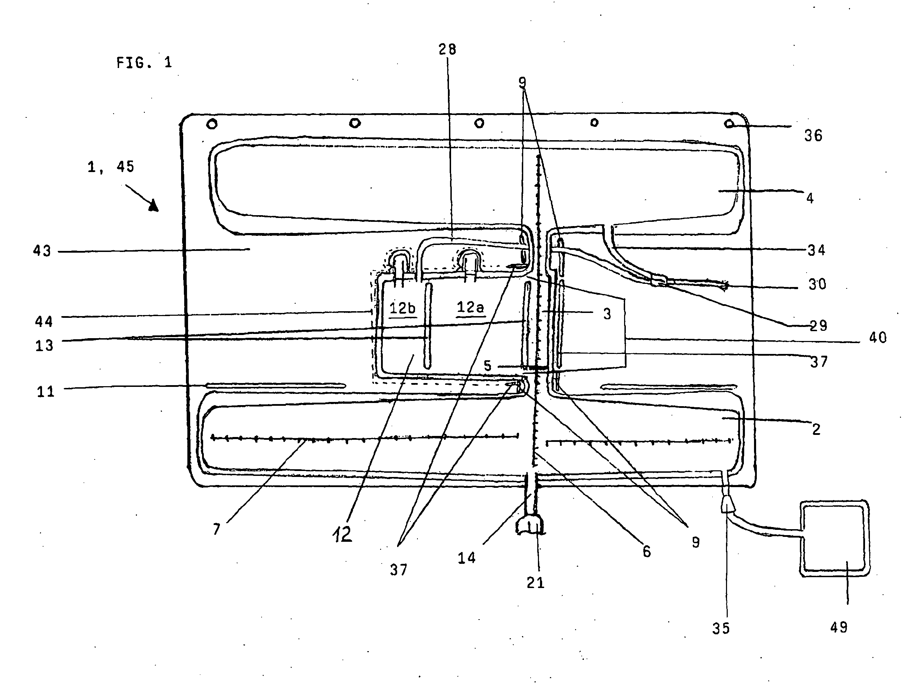

Die Fig. 1 bis 8 zeigen einen Abtrennbehälter 1 für eine Blutzentrifuge mit einem Fußabschnitt 2 zur Aufnahme von Erythrozyten 64, mit einem Mittelabschnitt 3 zur Aufnahme von Buffy-Coat 65 und mit einem Kopfabschnitt 4 zur Aufnahme von Blutplasma 66, wobei der Mittelabschnitt 3 eine geringere Querschnittsabmessung aufweist als Kopf- und Fußabschnitt 2, 4, wobei die Abschnitte 2, 3, 4 strömungsverbindbar sind und wobei der Fußabschnitt 2 vom Mittelabschnitt 3 abtrennbar ist.

Erfindungsgemäß ist der Fußabschnitt 2 bezüglich seines Aufnahmevolumens an das nach der Zentrifugierung zu erwartende Packvolumen der Erythrozyten 64 so anpassbar, dass das zu erwartende Packvolumen weitgehend vom Fußabschnitt 2 vollständig aufnehmbar ist und hierdurch die Phasengrenze 5 zwischen Erythrozyten 64 und Buffy-Coat 65 in einem zum Fußabschnitt 2 benachbarten Bereich des Mittelabschnittes 3 verläuft.

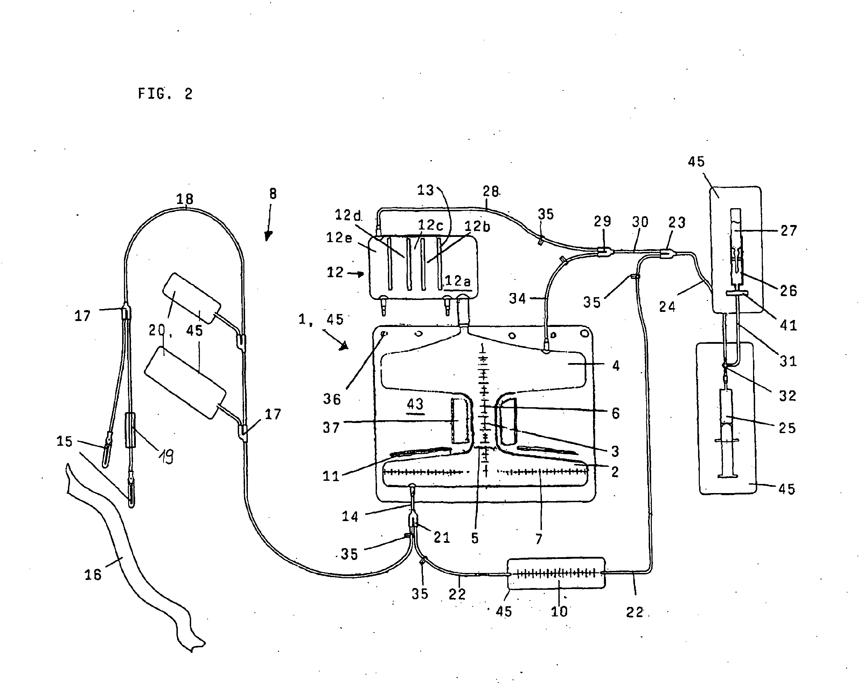

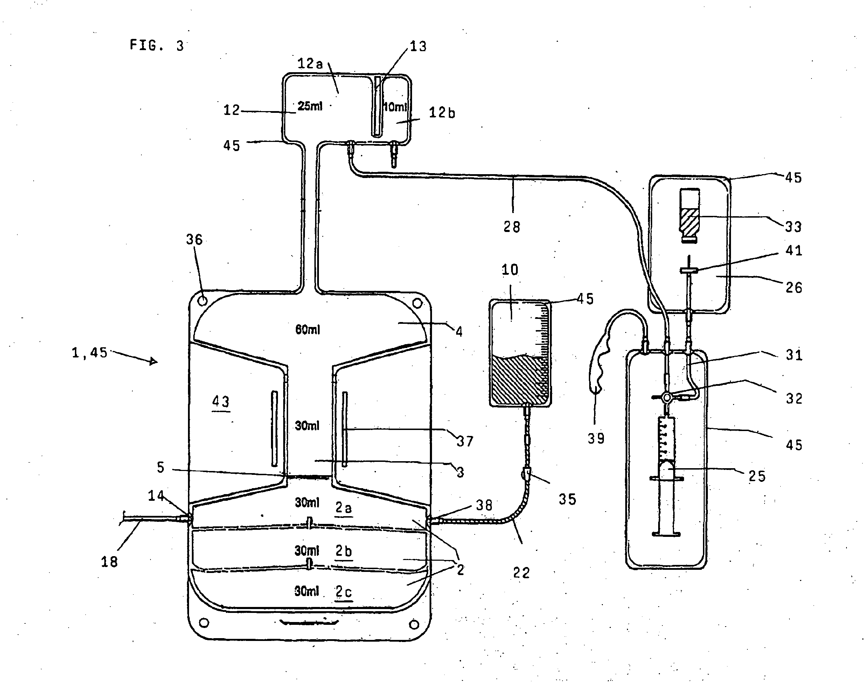

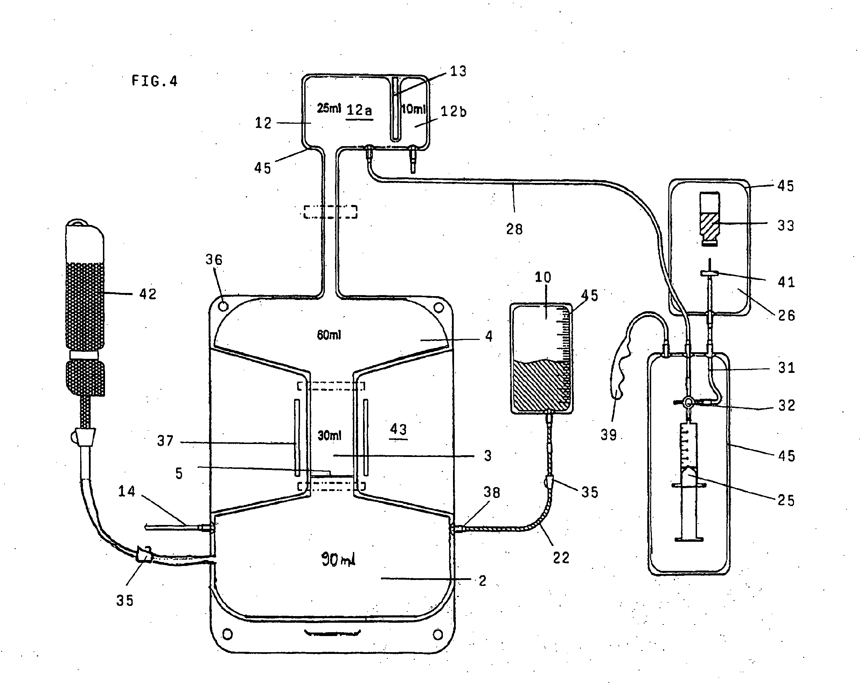

Am Abtrennbehälter 1 ist gemäß dem ersten und zweiten Ausführungsbeispiel des Abtrennbehälters 1 eine über alle Abschnitte 2, 3, 4 reichende vertikale Skalierung 6 zur Bestimmung des Füllvolumens des zuzuführenden Blutes vorgesehen. In Fig. 2 ist ein erstes Beispiel eines erfindungsgemäßen Systems gezeigt, dass eine Zuführeinrichtung 8 für Blut umfasst, über die das Blut zum Abtrennbehälter 1 gelangt. Nicht nur die Blutmenge, sondern auch die Zuführung der Substanz HES, die in Fig. 2 aus der Zuführeinrichtung 10 bzw. Vorratsbehälter 10 volumengenau zugegeben wird, wird über die Skalierung 6 überwachbar. In den Fig. 3 und 4 wird HES unter Ausnutzung der Gravitation aus dem Vorratsbehälter 10 zugeführt. Am Fußabschnitt 2 ist eine horizontale Skalierung 7 zur Voreinstellung des Aufnahmevolumens des Fußabschnittes 2 entsprechend dem nach der Zentrifugierung zu erwartenden Packvolumens der Erythrozyten 64 vorgesehen.

Bei dem in den Fig. 3 und 4 gezeigten dritten und vierten Ausführungsbeispiel des erfindungsgemäßen Abtrennbehälters 1 sind Volumenangaben auf den Abtrennbehälter 1 aufgedruckt. Hier sind die Volumen durch die Abschnitte 2, 3, 4, bereits vorbestimmt. Besonderheit beim dritten Ausführungsbeispiel ist gezeigt, dass dort der Fußabschnitt 2 in drei Kompartimente 2a, 2b, 2c unterteilt ist, durch die das Volumen vergrößert werden kann, wenn die innenliegenden, hier nicht näher bezeichneten Abbrechventile geöffnet werden. Im fünften Ausführungsbeispiel des Abtrennbehälters 1 sind die Skalen an Fuß- und Mittelabschnitt 2, 3 aus Vereinfachungsgründen nicht dargestellt.

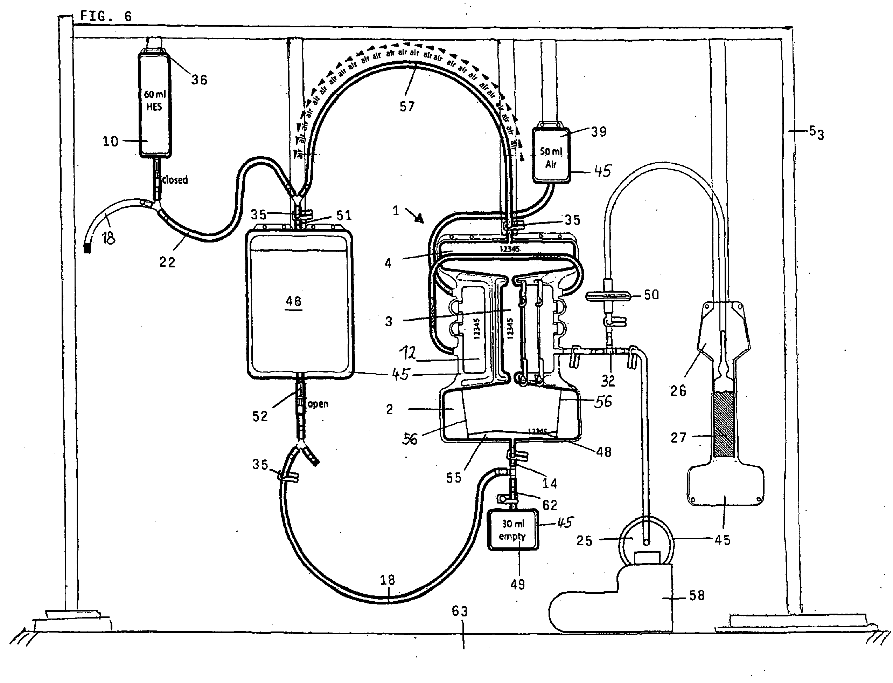

Zur Anpassung des Aufnahmevolumens des Fußabschnittes 2 an das zu erwartende Packvolumen der Erythrozyten 64 können an den Abtrennbehälter 1 gemäß Fig. 1 und 2 über die Aussparungen 11 zur Verkleinerung des Aufnahmevolumens Klemmvorrichtungen am Fußabschnitt 2 angebracht werden. Beim fünften Ausführungsbeispiel erfolgt gemäß Fig. 6 ein Abtrennen der nicht erforderlichen Bereiche durch Schweißnähte 56. Beim ersten und fünften Ausführungsbeispiel ist vorsorglich ein Aufnahmebehälter 49 für überschüssige Erythrozyten 64 vorgesehen, falls die Phasengrenze 5 unerwartet hoch im Mittelabschnitt 3 ausgebildet wird und nicht - wie gewünscht - in nächster Nähe zum Fußabschnitt 2. Auch der Abzug kleinster Mengen an Erythrozyten 64 kann hilfreich sein, wenn es um die korrekte Ausbildung und Befüllung des Einfrierbehälters 12 geht.

Der Abtrennbehälter 1 gemäß Fig. 3 weist bezüglich seines Fußabschnittes 2 zur Vergrößerung des Aufnahmevolumens strömungsverbindbare Kompartimente 2a, 2b, 2c auf. Zur Verkleinerung des Aufnahmevolumens können die die Kompartimente 2b, 2c abgeschweißt werden.

Eine Verkleinerung des Aufnahmevolumens des Fußabschnittes 2 findet gemäß Fig. 4 dadurch statt, dass eine Zuführeinrichtung 42 für Füllstoff vorgesehen ist, die mit dem Fußabschnitt 2 strömungsverbindbar ist, so dass zur Verkleinerung des Aufnahmevolumens des Fußabschnittes 2 Füllstoff in diesen einbringbar ist. Der Füllstoff liegt hier in Form von inerten Kügelchen vor.

Der Abtrennbehälter 1 ist aus zwei übereinanderliegenden Folien mit dreidimensionalen, hier durch Tiefziehen erhaltenen Bereichen für die Ausbildung der verschiedenen Abschnitte 2, 3, 4 ggf. des Einfrierbehälters 12 und einem dazwischenliegenden Blindbereich 43 gefertigt, wobei der Blindbereich 43 vorzugsweise flach und verschweißt ist. Kopfabschnitt 4 und Fußabschnitt 2 weisen zum Mittelabschnitt 3 geneigte Innenflächen auf. Am Rand des Blindbereichs 43 des Abtrennbehälters 1 sind Befestigungsmittel 36 zum Halten des Abtrennbehälters 1 in einem stabilen Einsatz vorgesehen, der in einen Zentrifugenbecher 59 einsatzbar ist. Die Befestigungsmittel 36 liegen hier in Form von Durchtrittsöffnungen vor, in die auch Laborklemmen eines Stativs 53 eingreifen können, wie bspw. in Fig. 6 dargestellt.

Alle Abtrennbehälter 1 umfassen einen Einfrierbehälter 12. Beim ersten und fünften Ausführungsbeispiel ist der Einfrierbehälter 12 integraler Bestandteil des Mittelabschnittes 3. Wenn der Mittelabschnitt 3 mit dem begehrten Buffy-Coat 65 gefüllt ist, werden beim ersten Ausführungsbeispiel die Bereiche 40, die bis dahin verschlossen waren, geöffnet, so dass der Mittelabschnitt 3 und der Einfrierbehälter 12 zunächst im Hinblick auf das Kompartiment 12a vereint sind und sich das Buffy-Coat 65 zunächst im Kompartiment 12a des Einfrierbehälter 12 ausbreitet. Die Öffnung der Bereiche 40 erfolgt erst nach der Zentrifugierung und erst dann, wenn Fuß- und Kopfabschnitt 2, 4, vom Mittelabschnitt 3 wirksam abgetrennt sind: Zur Unterstützung der Abtrennung von Kopf- und Fußabschnitt 2, 4 sind beim ersten Ausführungsbeispiel im Blindfeld 43 Aussparungen 9 vorgesehen, durch die eine Klemmvorrichtung geführt werden kann. Wenn der Einfrierbehälter 12 mit Buffy-Coat 65 und ggf..weiteren Substanzen nach Öffnen der Bereiche 40 wunschgemäß befüllt ist, wird er verschlossen und teilweise entlang einer Perforationslinie 44 aus dem Blindbereich 43 herausgetrennt, teilweise herausgeschnitten oder -geschweißt.

Die Figuren 2 bis 4 zeigen einen Einfrierbehälter 12, der dem Abtrennbehälter 1 zugeordnet und mit diesem strömungsverbindbar ist. Bei dem zweiten bis vierten Ausführungsbeispiel ist der Einfrierbehälter 12 am Kopfabschnitt 4 des Abtrennbehälters 1 angeordnet - hier werden zwei Zentrifugierungen in entgegengesetzter Richtung durchgeführt.

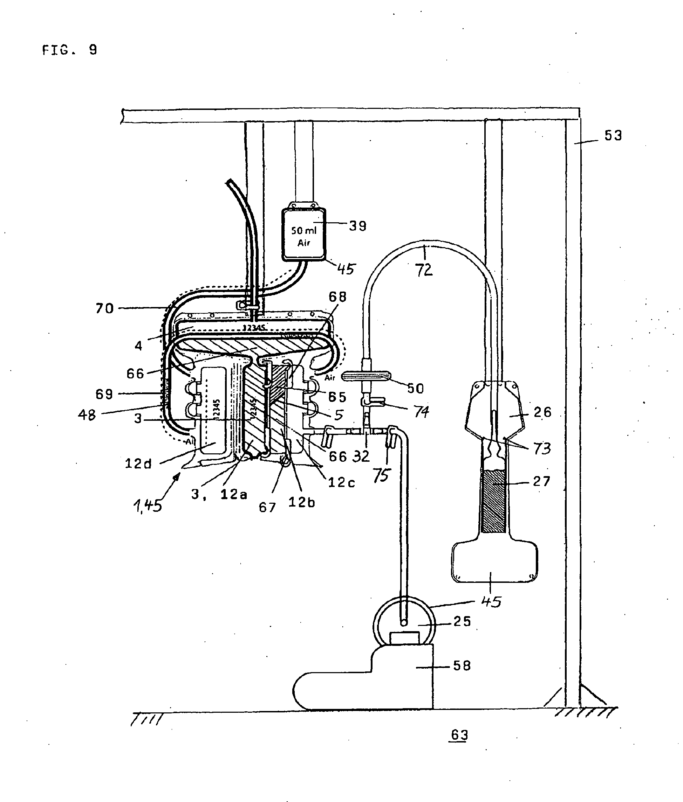

Bei den ersten vier Ausführungsbeispielen weist der Einfrierbehälter 12 strömungsverbindbare Kompartimente 12a, 12b, in Fig. 2 noch 12c, 12d, 12e, auf, zwischen denen ein Steg 13 oder mehrere Stege 13 eingearbeitet sind. Je nach erhaltener Menge an Buffy-Coat im Mittelabschnitt 3 kann in Fig. 2 bspw. das Kompartiment 12e des Einfrierbehälters 12 zur Verkleinerung des Aufnahmevolumens abgetrennt werden. Bei der Verkleinerung des Aufnahmevolumens ist die Zugabe weiterer Substanzen zu berücksichtigen. Beim fünften Ausführungsbeispiel, insbesondere gemäß Fig. 9, weist der Einfrierbehälter 12 die Kompartimente 12a, 12b, 12c und 12d auf, wobei Kompartiment 12a dem Mittelabschnitt 3 des Abtrennbehälters 1 entspricht.

Am Fußabschnitt 2 ist ein steril konnektierbarer Anschluss 14 zur Zugabe von Blut vorgesehen. Bei den ersten vier Ausführungsbeispielen wird HES über die HES-Leitung 22 und über Anschluss 14 oder 38 in den Fußabschnitt 2 geleitet. Beim fünften Ausführungsbeispiel wird HES in einen vorgeschalteten Blutsammelbehälter 46 eingeleitet.

Figur 2 zeigt den Abtrennbehälter 1 eingebunden in das erfindungsgemäße System zum Zeitpunkt der Blutentnahme am Patienten, das die Blutzuführung, die spätere Zentrifugierung und die spätere Präparation des Buffy-Coats im Einfrierbehälter 12 sowie die schrittweise Eliminierung aller Systembestandteile bis auf den Einfrierbehälter 12 beinhaltet. Die mit 8 bezeichnete Zuführeinrichtung zur Zugabe des Blutes umfasst zwei Kanülen 15 zur Entnahme von Blut aus der Nabelschnur 16, die jeweils eine perforierbare sterilisierte, hier nicht näher bezeichnete Latexumhüllung aufweisen. Die Kanülen 15 bilden ausgehend von einem Y-Stück 17 die Enden einer Verbindungsleitung 18. Mit 19 ist ein Kanülenschutz zum Schutz der zweiten Kanüle 15 bezeichnet. An der Verbindungsleitung 18 sind noch zwei weitere Y-Stücke 17 vorgesehen, über die Zuführeinrichtungen 20 für Antikoagulans gegen Blutgerinnung an die Verbindungsleitung 18 angeschlossen sind. Mit seinem den Kanülen 15 gegenüberliegenden Ende mündet die Verbindungsleitung 18 in einem weiteren Y-Stück 21, über das die Zufuhr über den Anschluss 14 in den Abtrennbehälter 1 erfolgt.

Über den zweiten Anschluss des Y-Stücks 21 führt die HES-Leitung 22 des Vorratsbehälters 10 bzw. der Zuführeinrichtung 10 für HES zum Anschluss 14. Das andere Ende der HES-Leitung 22 mündet in ein weiteres Y-Stück 23, das über eine Verbindungsleitung 24 zu einer Spritze 25 führt, über die der Substanztransport und ggf. auch Mischvorgänge und die Dosierung stattfinden und die auch für die Bewegung, das Pumpen von DMSO und Blutplasma 66 zuständig ist.

In den Fig. 3 und 4 wird HES direkt über den eigenen Anschluss 38 in den Fußabschnitt 2 eingespeist.

Der Einfrierbehälter 12 ist bei den ersten zwei Ausführungsbeispielen des Abtrennbehälters 1 über die Verbindungsleitung 28 indirekt mit einer Zuführeinrichtung 26 für DMSO verbunden, welche in Fig. 2 eine Abbrechampulle 27 mit DMSO und einen Partikelfilter 41 zum Zurückhalten von Bruch umfasst. Über ein weiteres Y-Stück 29, ein Verbindungsleitungstück 30, das Y-Stück 23 und die Verbindungsleitung 24 besteht eine Strömungsverbindung zur Spritze 25, die mit der Zuführeinrichtung 26 für DMSÖ über eine Verbindungsleitung 31 in Verbindung steht und DMSO über die entsprechende Stellung des Mehrwegehahns 32 zum Einfrierbehälter 12 per Kolbenbewegung transferieren kann. Ein Mehrwegehahn 32 ist auch im fünften Ausführungsbeispiel vorgesehen.

Bei den Figuren 3 und 4 ist der Anschluss des Einfrierbehälters 12 zur Zuführeinrichtung für DMSO ebenfalls über eine Verbindungsleitung 28 realisiert, der zur Spritze 25 mit Mehrwegehahn 32 führt. Die dortige Verbindung 31 führt zu einer Durchstechampulle 33 mit DMSO, die mittels nicht näher bezeichneter Kanüle aufgestochen wird.

Der Kopfabschnitt 4 des Abtrennbehälters 1 ist in Fig. 2 mit einer Entnahmeeinrichtung zur Entnahme von Blutplasma verbunden. Die Entnahmeeinrichtung wird durch die Spritze 25 repräsentiert, die über Verbindungsleitung 34, Y-Stück 29, Schlauchstück 30, Y-Stück 23, Verbindungsleitung 24 und Mehrwegehahn 32 mit dem Kopfabschnitt 4 verbunden ist. Die Zuführeinrichtung 10 für HES, die Zuführeinrichtung 26 für DMSO und die Spritze 25 nebst allen Verbindungsleitungen 28, 24, 31, HES-Leitung 22, Verbindungsleitungsstück 30, Y-Stücken 23, 29, und Schlauchklemmen 35 zur Unterbrechung der Strömungsverbindung sowie der Abtrennbehälter 1 und der Einfrierbehälter 12 sowie ggf. die Zuführeinrichtung 42 für Füllstoff in Fig. 4 sind Bestandteile eines geschlossenen Systems. Die Schließung des Systems erfolgt nach der Befüllung des Abtrennbehälters 1 mit Blut über die Verbindungsleitung 18 und den Anschluss 14.

Die Geschlossenheit des Systems wird dadurch hergestellt, dass alle Bestandteile, der Abtrennbehälter 1, die Spritze 25, die DMSO-Zuführeinrichtung 26, die Zuführeinrichtung 10 für HES, der Einfrierbehälter 12, ggf. die Zuführeinrichtung 42 für Füllstoff (Fig. 4), ggf. den Aufnahmebehälter 49 für Erythrozyten (Fig. 1), ggf. Druckausgleicheinrichtung 39 (Fig. 3 bis 11) sind in einem ummantelnden / umhüllenden Behältnis 45 angeordnet und sind sterilisiert. Zum einen umhüllt das Behältnis 45 eine Substanz - wie Blut, dessen Bestandteile, HES -, zum anderen die Spritze 25, das Mehrwegehahn 32, die Abbrechampulle 27, den Partikelfilter 41, ggf. die Durchstichampulle 33 (Fig. 3, 4). Die Verbindungsleitungen 28, 24, 31, die HES-Leitung 22, das Verbindungsleitungsstück 30 sind untereinander und mit den jeweiligen Behältnissen 45 der Spritze 25, der DMSO-Zuführeinrichtung 26, des Vorratsbehälters bzw. der Zuführeinrichtung 10 für HES, des Abtrennbehälters 1 und des Einfrierbehälters 12 steril verbunden. Die Spritze 25, der Mehrwegehahn 32 und die Abbrechampulle 27, ggf. die Durchstichampulle 33, sind von außen, ohne Öffnung des Behältnisses 45, also unter Beibehalt der Wandung des Behältnisses 45 betätigbar. In dem den Abtrennbehälter 1 ausbildenden Behältnis 45 sind Stützelemente 37 zur Stabilisierung des Mittelabschnittes 3 enthalten. Über den Mehrwegehahn 32 der Spritze 25 wird überschüssige Luft in das die Spritze 25 ummantelnde, beutelartige Behältnis 45 entlassen. In Fig. 4 sind mittels Strichlinie Klemmvorrichtungen angedeutet, die die Abschnitte 2, 3, 4 und den Einfrierbehälter voneinander trennen können, was insbesondere vor der ersten Zentrifugierung zwischen Einfrierbehälter 12 und Kopfabschnitt 3 erforderlich ist und was nach dem Zentrifugieren zur Vermeidung unerwünschter Mischungen zumindest zwischen Fußabschnitt 2 und Mittelabschnitt 3 erforderlich ist.

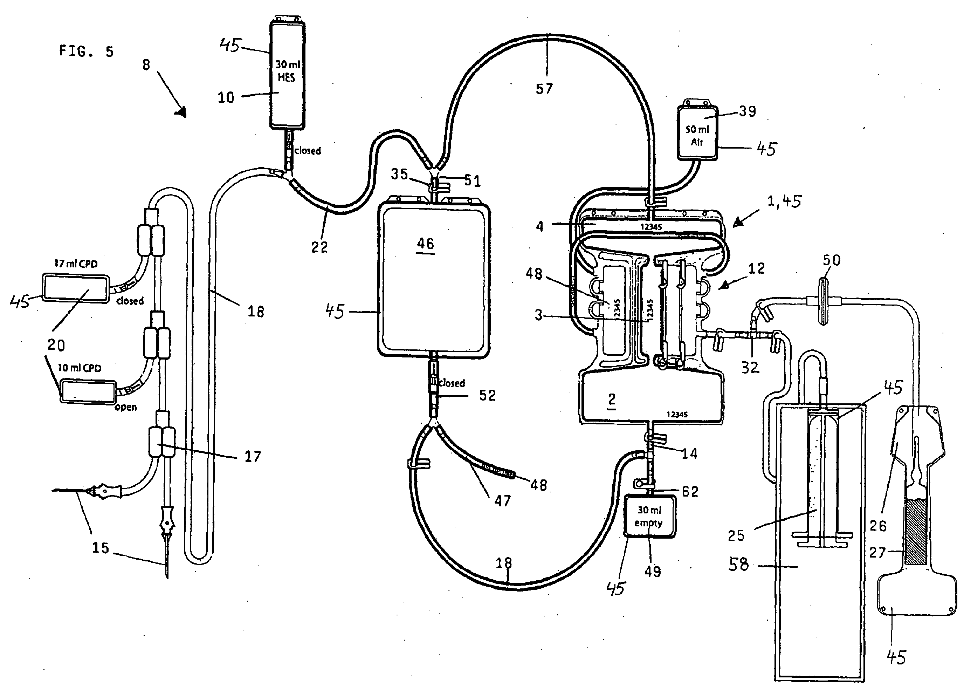

Die Spritze 25 gemäß Fig. 3 und 4 sowie gemäß Fig. 5 bis 10 transportiert, pumpt und dosiert nur DMSO, ggf. enthalten in einer kryoprotektiven Mischung. Ummantelnde Behältnisse 45 aller Komponenten sind dort ebenfalls vorgesehen, so dass auch dort geschlossene Komponenten innerhalb eines geschlossenen Systems miteinander kontaminationsfrei korrespondieren. Über den Mehrwegehahn 32 wird überschüssige Luft in den fig. 3 und 4 in eine Druckausgleicheinrichtung 39 entlassen. Beim fünften Ausführungsbeispiel sind der Mehrwegehahn 32 und der Rundfilter 50 zwar nicht von einem Behältnis 45 umgeben, sind aber dennoch steril gegen die Umwelt abgedichtet.

Auch die Zuführeinrichtung 8 für Blut bietet nicht viel Angriffsfläche für einen Austausch mit der Umwelt. So sind auch die beiden Zuführeinrichtungen 20 für Antikoagulans gemäß den Fig. 2, 5 in Behältnissen 45 enthalten und mit der Verbindungsleitung 18 steril verbunden. Einzige Schwachstelle sind die Kanülen 15, die aber das bis zur Entnahme des Nabelschnurblutes aus der Nabelschnur 16 geschlossene System nur sehr kurzzeitig unter Perforierung der Latexhülle öffnen. Mit 19 ist ein Kanülenschutz bezeichnet, der vor und nach Ingebrauchnahme zum Schutz vor Verletzungen der medizinischen Fachkraft über die Kanüle 15 gezogen ist.

Ein fünftes Ausführungsbeispiel, das sowohl den erfindungsgemäßen Abtrennbehälter 1 als auch das erfindungsgemäße System gemäß einem zweiten Beispiel zeigt, ergibt sich aus den Figuren 5 bis 12.

Fig. 5 zeigt das System, so wie es kurz nach der Blutentnahme aus der nur aus Fig. 2 ersichtlichen Nabelschnur 16 vorliegt. Das System beinhaltet wie bei Fig. 2 die Blutzuführung, die spätere Zentrifugierung / Fraktionierung und die spätere Präparation des Buffy-Coats im Einfrierbehälter 12 sowie die schrittweise Eliminierung aller Systembestandteile bis letztlich auf den Einfrierbehälter 12. Die mit 8 bezeichnete Zuführeinrichtung für Blut umfasst zwei Kanülen 15 zur Entnahme von Blut aus der hier nicht gezeigten Nabelschnur, eine Zuführeinrichtung 10 für HES, einen Blutsammelbehälter 46 und einen Probenahmeabschnitt 47 mit einer patientenbezogenen Identifikationsnummer 48.

Die Kanülen 15 bilden ausgehend von einem Y-Stück 17 die Enden einer Verbindungsleitung 18. An der Verbindungsleitung 18 sind noch zwei weitere Y-Stücke 17 vorgesehen, über die Zuführeinrichtungen 20 für Antikoagulans gegen Blutgerinnung an die Verbindungsleitung 18 angeschlossen sind. Zunächst werden 17 ml Antikoagulans aus der oberen Antikoagulans-Zuführeinrichtung 20 in den Blutsammelbehälter 46 geleitet. Die Zufuhr erfolgt über die Schlauchklemme 35 am oberen Anschluss 51 des Blutsammelbehälters 46. Danach erfolgen die Punktierung der Nabelschnur 16 und die Blutzuführung in den Blutsammelbehälter 46. Der Transport wird ermöglicht durch die pulsierende Pumptätigkeit des Blutes, die unterstützt werden kann, indem das System unterhalb der Einstichstelle platziert wird und so die Gravitation ausgenutzt werden kann. Nachdem der Blutsammelbehälter 46 nahezu vollständig gefüllt ist, erfolgt eine zweite Zugabe von 10 ml Antikoagulans aus der unteren Zuführeinrichtung 20, mit dem nun die Verbindungsleitung 18 gereinigt wird und alles Blut ohne nennenswerte Verluste an Nabelschnurblut in den Blutsammelbehälter 46 gelangt. Nachdem die Befüllung des Blutsammelbehälters 46 abgeschlossen ist, wird die Verbindungsleitung 18 kurz vor der Zuführeinrichtung 10 von HES steril abgeschweißt und somit werden die Kanülen 15 und die Antikoagulans-Zuführeinrichtungen 20 eliminiert. Das System ist nun geschlossen und wird - auch wenn weitere Systembestandteile abgetrennt werden - nicht mehr geöffnet, bis jemand Buffy-Coat 65 aus dem Einfrierbehälter 12 benötigt. Das bereits etwas reduzierte System wird nun vom Krankenhaus zur Blutbank transportiert.

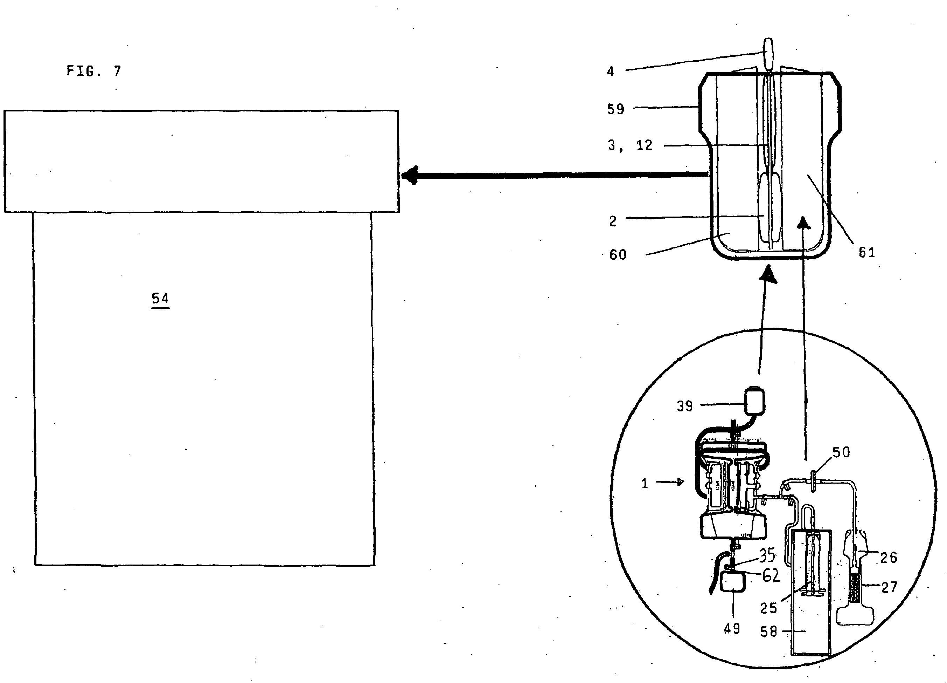

In der Blutbank wird das System mit Ausnahme der Spritze 25 an einem in Fig. 6 gezeigten Stativ 53 an Befestigungen 36 an den Behältnissen 45 aufgehängt und es wird der Anschluss 52 des Blutsammelbehälters 46 geöffnet um den mit einer Identifikationsnummer 48 gekennzeichneten Probenahmeabschnitt 47 mit Blut zu befüllen. Zur Befüllung des Probenahmeabschnitts 47 mit Blut wird dieser mittels eines herkömmlichen Rollwerkzeuges in Richtung Anschluss 52 von Luft leergerollt bzw. entlüftet. Sodann saugt sich der leergerollte Probnahmeabschnitt 47 voll Blut aus dem Blutsammelbehälter 46. Die Probenahme erfolgt somit ohne das System zu öffnen. Erstmals wird eine Befreiung von Luft aus einer Leitung per Rollwerkzeug praktiziert. Nach Befüllung des Probenahmeabschnitts 47 mit Blut wird der Anschluss 52 geschlossen und der Probenahmeabschnitt 47 abgeschweißt, um diesen mit dem Blut einer in Fig. 7 gezeigten Zentrifuge 54 zuzuführen und den Hämatokritwert zu ermitteln, woraus sich dann Rückschlüsse auf das Packvolumen und den benötigten Platz im Fußabschnitt 2 und dessen Manipulation ziehen lassen.

In Fig. 6 ist das erfindungsgemäße System zu einem Zeitpunkt gezeigt, nachdem eine Manipulation des Volumens am Fußabschnitt 2 erfolgt ist. Am Fußabschnitt 2 ist nunmehr nur das Volumen zwischen den zur Manipulation des Volumens angebrachten Schweißnähten 56 als. Aufnahme für die in der bevorstehenden Zentrifugierung zu erhaltenden Erythrozyten 64 vorgesehen.

In Fig. 6 wurde bereits begonnen, den Abtrennbehälter 1 ausgehend vom Blutsammelbehälter 46 und dessen offenen Anschluss 52 sowie die ebenfalls mit 18 bezeichneten Verbindungsleitung mit Blut 55 über den Fußabschnitt 2 und den dortigen Anschluss 14 zu befüllen. Der Blutsammelbehälter 46 wird entleert und über den in Bezug auf eine Luftleitung 57 geöffneten oberen Anschluss 51 mit Luft befüllt, die vom Abtrennbehälter 1 kommt. Es findet quasi eine Zirkulation statt, wobei die Luft aus dem Abtrennbehälter 1 mit zunehmender Befüllung mit Blut aus dem Blutsammelbehälter 46 in letzteren verdrängt wird. Die Luftleitung 57 ist mit dem Kopfabschnitt 4 verbunden und wird dann mittels Schlauchklemme 35 geschlossen, wenn das Blut aus dem Blutsammelbehälter 46 über die Verbindungsleitung 18 kommend bis zum oberen Rand des Kopfabschnitts 4 gestiegen ist. Pfeile mit der Bezeichnung "air" verdeutlichen die Richtung der Luftströmung in der Luftleitung 57.

Aus Fig. 6 geht weiter hervor, dass im fünften Ausführungsbeispiel die Spritze 25 auch mittels Pumpe 58 bedienbar ist, was insbesondere bei der DMSO-Zugabe eine Rolle spielt. Die Befüllung der Spritze 25 mit DMSO erfolgt dagegen manuell. Zudem ist der Abtrennbehälter 1 an seinen Abschnitten 2, 3, 4 und dem Einfrierbehälter 12 mit der Identifikationsnummer 48 gekennzeichnet und es stehen eine Druckausgleicheinrichtung 39 für den Einfrierbehälter 12 sowie ein Aufnahmebehälter 49 für eventuelle überschüssige Erythrozyten 64 nach Zentrifugierung zur Verfügung.

Nachdem der Abtrennbehälter 1 mit Blut 55 befüllt ist, bleibt die Luftleitung 57 zunächst noch geöffnet und es wird auch eine Strömungsverbindung über die HES-Leitung 22 zwischen der Zuführeinrichtung 10 für HES und dem inzwischen geleerten Blutsammelbehälter 46 hergestellt, der seinerseits noch in Strömungsverbindung zum Abtrennbehälter 1 steht. HES gelangt also ebenfalls über den Blutsammelbehälter 46, die Verbindungsleitung 18 und den Anschluss 14 und Fußabschnitt 2 in den Abtrennbehälter 1 und nimmt dabei alle Reste des wertvollen Blutes 55 mit. Wenn die HES-Zufuhr abgeschlossen ist, wird der Abtrennbehälter 1 über Schlauchklemmen 35 verschlossen. Das System wird weiter verkleinert, indem der Blutsammelbehälter 46 und damit auch die Zuführeinrichtung 10 für HES an anschlussnahen Bereichen der Luftleitung 57 und der Verbindungsleitung 18 steril abgeschweißt wird.

In Fig. 7 ist gezeigt, dass das verbleibende System in einen Zentrifugenbecher 59 eingebracht wird. Der Zentrifugenbecher 59 weist zwei Innenbehälter 60 und 61 auf. Der Innenbehälter 61 nimmt die ummantelte Spritze 25, die Pumpe 58 und die Zuführeinrichtung 26 für DMSO auf. Der Innenbehälter 60 kann zum Gewichtsausgleich, zur Herstellung einer Balance mit Füllstoff (Feststoff oder Flüssigkeit) befüllt und verschlossen werden. Die einander zugewandten Außenflächen der Innenbehälter 60, 61 bilden eine weitgehend an die Formgebung des Abtrennbehälters 1 angepasste Aufnahme aus und ermöglichen außerdem die Unterbringung der Druckausgleicheinrichtung 39 und des Aufnahmebehälters 49, dessen Anschluss 62 durch eine Schlauchklemme 35 beim Zentrifugieren verschlossen ist. Der Zentrifugenbecher 59 wird mit einem hier nicht dargestellten Deckel so verschlossen, dass auch der Kopfabschnitt 4 nicht beschädigt wird. Schließlich wird der Zentrifugenbecher in die Zentrifuge 54 eingebracht und es wird zentrifugiert.

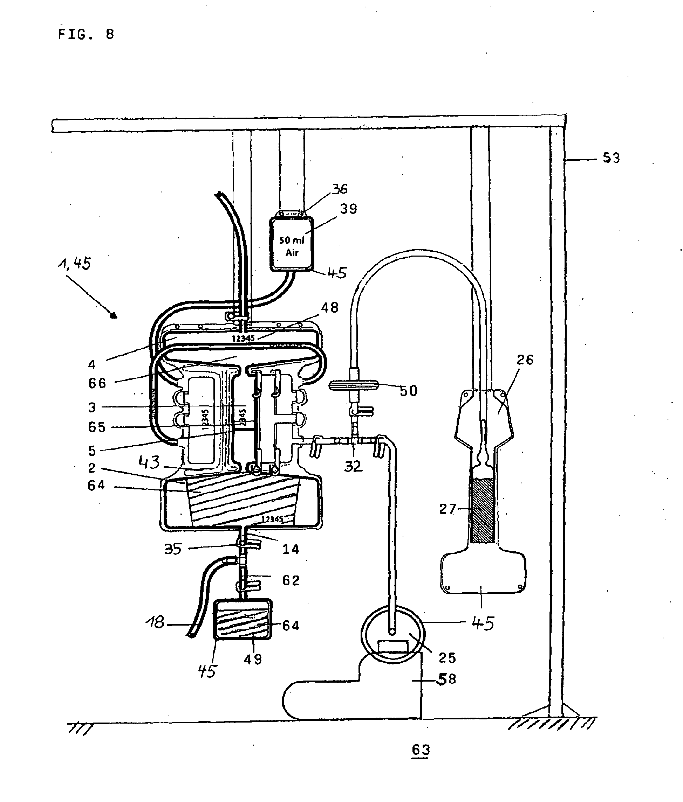

In Fig. 8 ist dargestellt, dass das System nach dem Zentrifugieren aus der Zentrifuge 54 entnommen und wieder am Stativ 53 befestigt bzw. auf der Unterlage 63 aufgelegt wird. Die Phasengrenze ist mit 5 bezeichnet und erstreckt sich quer über den Mittelabschnitt 3, ein wenig unterhalb der Hälfte des Mittelabschnitts 3 über der Phasengrenze 5 erstreckt sich das Buffy-Coat 65, darüber das Blutplasma 66. An und für sich war der Bereich unterhalb der Phasengrenze 5 und der Fußabschnitt 2 mit Erythrozyten 64 befüllt, die hier in Fig. 8 aber bereits über den Fußabschnitt 2 aus dem Mittelabschnitt 3 in den Aufnahmebehälter 49 für Erythrozyten 64 entlassen worden sind, um den Bereich des Einfrierbehälters 12 weitgehend frei von Erythrozyten 64 zu halten. Die Kapazität des Aufnahmebehälters 49 ist hier auf 30 ml festgelegt und großzügig abgestimmt auf die erwartete Menge an - hier schraffiert dargestellten - Erythrozyten 64 aus dem Mittelabschnitt 3. In Fig. 8 ist also ein Teil der Erythrozyten 64 in den Aufnahmebehälter 49 entlassen, so dass die Erythrozyten 64 im Abtrennbehälter 1 nur noch im Fußabschnitt 2 anzutreffen sind und der Einfrierbehälter 12 mit dem integrierten Mittelabschnitt 3 sowie der Kopfabschnitt 4 mit dem Blutplasma 66 weiteren Arbeitsschritten zugeführt werden können.

Der mit einer Identifikationsnummer 48 gekennzeichnete Fußabschnitt 2 und der Aufnahmebehälter 49 werden in einem nächsten Schritt vom übrigen System steril abgetrennt. Der Abtrennbehälter 1 besteht aus einem Behältnis 45, der aus übereinanderliegenden Folien gebildet wurde und verschiedene Kammern aufweist, die untereinander verbindbar und abtrennbar sind und eben weist auch Blindfelder 43 auf. Die Blindfelder 43 werden aus direkt aufeinanderliegenden - kammerfrei - verbundenen Folienabschnitten gebildet und ermöglichen ein einfaches steriles Abtrennen, Abschweißen von Abschnitten des Abtrennbehälters 1. Die Verbindung des Mittelabschnitt 3 zum Fußabschnitt 2 wird jedenfalls steril verschlossen, ohne dass es zur Öffnung des Systems kommt.

In Fig. 9 ist gezeigt, dass der Fußabschnitt 2 steril abgetrennt ist und das Buffy-Coats 65 vom Mittelabschnitt 3, der gleichzeitig das Kompartiment 12a des Einfrierbehälters 12 bildet, in ein weiteres Kompartiment 12b des Einfrierbehälters 12 überführt wurde. Dazu wird der untere Verschluss 67 zwischen den Kompartimenten 12a und 12b geöffnet und das Buffy-Coat 65 wird von dem vom Kopfabschnitt 4 kommenden Blutplasma 66 in das Kompartiment 12b verdrängt. Das Buffy-Coat 65 wird durch eine nach rechts aufsteigende Schraffur, das Blutplasma 66 durch eine nach links aufsteigende Schraffur verdeutlicht. Damit eingeschlossene Luft kein Hindernis bildet, wird der Verschluss 68 geringfügig geöffnet, so dass Luft durch das Kompartiment 12c in die Luftleitung 69 austreten kann. Bei dem vorliegenden fünften Ausführungsbeispiel mündet die ebenfalls mit der Identifikationsnummer 48 gekennzeichnete Verbindungsleitung 69 in das Kompartiment 12d des Einfrierbehälters 12. Von dort aus kann die verdrängte Luft über die Luftleitung 70 in den Druckausgleichbehälter 39 eintreten, der ein bestimmtes Aufnahmevolumen für die aus dem Einfrierbehälter 12 verdrängte Luft aufweist.

Wenn die Kompartimente 12a und 12b vollständig mit Buffy-Coat 65 und Blutplasma 66 gefüllt sind, wird die Verbindung zwischen Kopfabschnitt 4 und Mittelabschnitt 3 bzw. Kompartiment 12a geschlossen, hier verschweißt und der Kopfabschnitt 4 vollständig abgetrennt. Es erfolgt nun eine Vermischung von Buffy-Coat 65 und Blutplasma 66 und eine Verteilung auf die Kompartimente 12a, 12b und 12c. Die Verteilung kann per Hand durchgeführt werden. Unterstützung wird hier über eine Schüttelvorrichtung 71 realisiert. Die Verbindungsleitung 69 ist geschlossen.

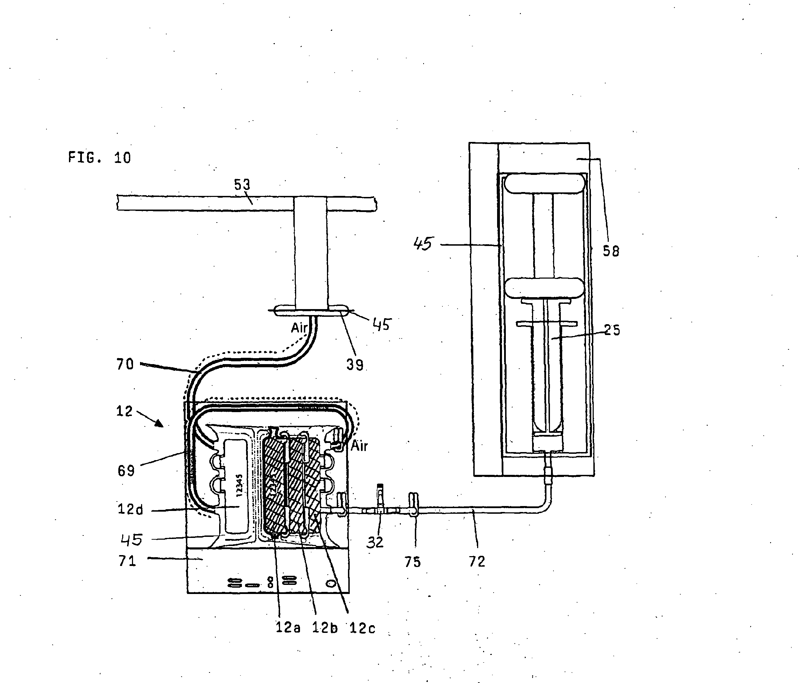

Wenn die Verteilung der Mischung aus Buffy-Coat 65 und Blutplasma 66 stattgefunden hat, was in Fig. 10 durch die Kreuzschraffur in den Kompartimenten 12a, 12b, 12c verdeutlicht ist, wird DMSO über die Pumpe 58 und die Spritze 25 zugegeben. Zuvor wird die Spritze 25 durch Aufziehen des Kolbens mit DMSO aus der Brechampulle 27 manuell aufgefüllt und dabei von außen, durch die Wandung des sie ummantelnden Behältnisses 45 hindurch betätigt. Die Brechampulle 27 wird zum Aufziehen der Spritze 25 zunächst innerhalb der Zuführeinrichtung 26 für DMSO aufgebrochen und nach oben verschoben, so dass das Ende der DMSO-Leitung 72 bis zum Boden der Brechampulle 27 reicht. Es sind innerhalb der Zuführeinrichtung 26 für DMSO Schultern 73 vorgesehen, die flexibel sind, jedoch ausreichend stabil, um die nach oben geschobene Brechampulle 27 zu fixieren. Die Schlauchklemmen 74, 75 sind entfernt und die Spritze 25 saugt das DMSO über die DMSO-leitung 72 und den in die passende Offenstellung verbrachten Mehrwegehahn 32 ein. Ein Rundfilter 50 hält Scherben der Brechampulle 27 zurück. Nach diesem Vorgang wird die Zuführvorrichtung 26 für DMSO vom übriggebliebenen System steril abgetrennt. Auch hier ist Sorge getragen, dass keine Öffnung des Systems stattfindet und somit in einem normalen Labor anstatt in einem teuren Reinraum gearbeitet werden kann.

In Fig. 10 ist das Restsystem nach Abtrennung der Zuführeinrichtung 26 für DMSO dargestellt. Es ist gezeigt, dass das DMSO von der Spritze 25 und der Pumpe 58 über die DMSO-Leitung 72 in den Einfrierbehälter 12, hier in das Kompartiment 12c, gelangt. Im fünften Ausführungsbeispiel wird nicht DMSO in seiner Reinform verwendet, sondern in einer Mischung von 50% DMSO und 50% einer 10%igen wässrigen Lösung aus "DEXTRAN 40". Die Spritze 25 wirkt bei der Befüllung des Einfrierbehälters 12 mit einer Pumpe 58 zusammen, die sicherstellt, dass die Befüllung langsam vor sich geht und ca. 10 min dauert.

In Fig. 10 ist weiter dargestellt, dass DMSO - hier innerhalb der Mischung - zunächst in Kompartiment 12c gelangt. Luft wird über die Verbindungsleitung 69 zum Kompartiment 12d verdrängt und gelangt von dort in die Druckausgleicheinrichtung 39. Der Einfrierbehälter 12 ist auf einer gekühlten Schüttelvorrichtung 71 angeordnet, die dafür sorgt, dass sich DMSO in allen drei Kompartimenten 12a, 12b, 12c gleichmäßig ausbreitet.

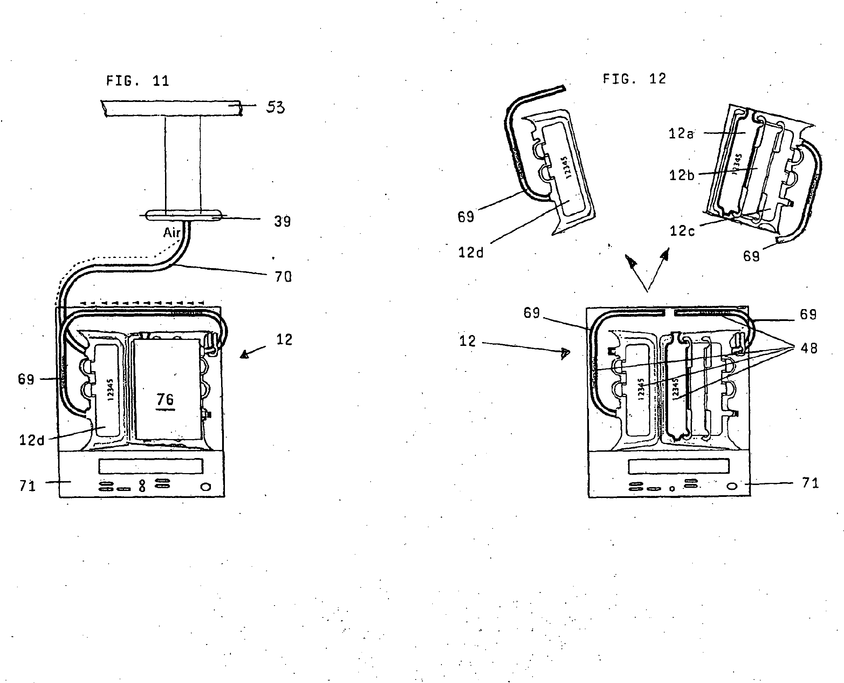

Fig. 11 zeigt, dass die in Fig. 10 noch erforderliche DMSO-Leitung 72 nun steril abgeschweißt ist. Das System beinhaltet nur noch den Einfrierbehälter 12 mit Verbindungsleitung 69, Luftleitung 70 und die Druckausgleicheinrichtung 39, die am Stativ 53 angeordnet ist. Der Einfrierbehälter 12 ist auf der Schüttelvorrichtung 71 angeordnet. Auf die Kompartimente 12a, 12b und 12c ist eine Matrize 76 aufgelegt, die an ihrer Unterseite eine an die Kompartimente 12a, 12b, 12c angepasste Formgebung aufweist, die jedoch so bemessen ist, dass ein Teil der Mischung aus Buffy-Coat 65, Blutplasma 66 und DMSO zur Verbindungsleitung 69 und in das noch leere Kompartiment 12d verdrängt wird. Auf die Kompartimente 12a, 12b, 12c wird Druck von oben ausgeübt wird und Kompartiment 12d wird befüllt. Die verdrängte Luft gelangt über die Luftleitung 70 in die Druckausgleicheinrichtung 39.

Nachdem die Verteilung der Mischung aus Buffy-Coat 65, Blutplasma 66 und DMSO stattgefunden hat und der gesamte Einfrierbehälter 12 gleichmäßig befüllt ist, wird die Matrize 76 entfernt und es wird die Luftleitung 70 vom Kompartiment steril abgeschweißt. In Fig. 12 ist außerdem gezeigt, dass die Verbindungsleitung 69 steril durchtrennt und die Enden steril verschlossen sind. Zudem wird in Fig. 12 die Unterteilung des Einfrierbehälters 12 in zwei Teile verdeutlicht. Ein erster Teil umfasst das Kompartiment 12d, ein zweiter Teil umfasst die Kompartimente 12a (Mittelabschnitt 3), 12b, 12c. Die beiden Teile können nun in verschiedenen Blutbanken gelagert werden und sind durch die Identifikationsnummern 48 stets zuordenbar. Die Buffy-Coat-Mischung kann dann bequem und kontaminationsfrei über die steril ummantelten, nicht näher bezeichneten Anschlüsse entnommen werden von denen jedes der beiden Teile zwei Stück aufweist. In den Fig. 7 und 8 sind die beiden Anschlüsse bezüglich des Teils, der die Kompartimente 12a, 12b, 12c umfasst so ausgestaltet, dass einer in das Kompartiment 12b öffnet und einer in das Kompartiment 12c. Die Reste der Verbindungsleitung 69 können zur Probenahme verwendet werden.

Hinsichtlich weiterer, in den Figuren nicht gezeigter Merkmale wird auf den allgemeinen Teil der Beschreibung verwiesen. Insbesondere sind bei den in den Fig. 1 bis 4 gezeigten Ausführungsbeispielen ebenfalls - hier nicht dargestellte - Druckausgleicheinrichtungen vorgesehen, damit verdrängte Luft im System bleibt und kein Austausch mit der Umwelt stattfindet.

Abschließend sei darauf hingewiesen, dass die erfindungsgemäße Lehre nicht auf die voranstehend erörterten Ausführungsbeispiele eingeschränkt ist.

## Patentansprüche

1. Verfahren zur Trennung von Blut, wobei verschiedene Blutfraktionen , nämlich Erythrozyten, Buffy-Coat und Blutplasma, gewonnen werden,
wobei Blut in einen Abtrennbehälter eingefüllt wird und dann in verschiedene, übereinandergeordnete, strömungsverbundene Abschnitte des Abtrennbehälters, nämlich Kopfabschnitt zur Aufnahme des Blutplasmas, Mittelabschnitt zur Aufnahme des Buffy-Coats, Fußabschnitt zur Aufnahme der Erythrozyten, zentrifugiert wird,
• wobei anhand des Hämatokritwertes des zugeführten Blutes das künftige Packvolumen der zu zentrifugierenden Erythrozyten bestimmt wird,
• wobei der Fußabschnitt in seinem Aufnahmevolumen an das nach der Zentrifugierung zu erwartende Packvolumen der Erythrozyten derart angepasst wird, dass die sich beim Zentrifugieren ausbildende Phasengrenze zwischen Buffy-Coat und Erythrozyten in einem zum Fußabschnitt benachbarten Bereich des Mittelabschnittes des Abtrennbehälters positioniert wird und
• wobei die Menge an zugeführtem Blut volumengenau, unter Berücksichtigung des zu erwartenden Packvolumens der Erythrozyten, in den Abtrennbehälter eingegeben wird,
**dadurch gekennzeichnet,**
**dass** nach der Zentrifugierung die Abtrennung des Fußabschnittes (2) vom Mittelabschnitt (3) erfolgt und dass der Mittelabschnitt (3) Teil eines Einfrierbehälters (12) ist, der während der Zentrifugierung vom übrigen Bereich des Einfrierbehälters (12) abgetrennt ist und dass nach der Zentrifugierung die Verbindung zum zuvor abgetrennten Bereich des Einfrierbehälters (12) hergestellt wird und das Buffy-Coat zumindest in Teile des gesamten Einfrierbehälters (12) gelangt.

2. Verfahren zur Trennung von Blut, wobei verschiedene Blutfraktionen, nämlich Erythrozyten, Buffy-Coat und Blutplasma, gewonnen werden,
wobei Blut in einen Abtrennbehälter eingefüllt wird und dann in verschiedene, übereinandergeordnete, strömungsverbundene Abschnitte des Abtrennbehälters, nämlich Kopfabschnitt zur Aufnahme des Blutplasmas, Mittelabschnitt zur Aufnahme des Buffy-Coats, Fußabschnitt zur Aufnahme der Erythrozyten, zentrifugiert wird,
• wobei anhand des Hämatokritwertes des zugeführten Blutes das künftige Packvolumen der zu zentrifugierenden Erythrozyten bestimmt wird,
• wobei der Fußabschnitt in seinem Aufnahmevolumen an das nach der Zentrifugierung zu erwartende Packvolumen der Erythrozyten derart angepasst wird, dass die sich beim Zentrifugieren ausbildende Phasengrenze zwischen Buffy-Coat und Erythrozyten in einem zum Fußabschnitt benachbarten Bereich des Mittelabschnittes des Abtrennbehälters positioniert wird und
• wobei die Menge an zugeführtem Blut volumengenau, unter Berücksichtigung des zu erwartenden Packvolumens der Erythrozyten, in den Abtrennbehälter eingegeben wird,
**dadurch gekennzeichnet,**
**dass** nach der Zentrifugierung die Abtrennung des Fußabschnittes (2) vom Mittelabschnitt (3) erfolgt und dass nach der Zentrifugierung und nach Abtrennung des Fußabschnittes vom Mittelabschnitt eine Strömungsverbindung zwischen einem dem Abtrennbehälter (1) zugeordneten und mit diesem strömungsverbindbaren Einfrierbehälter (12) und dem Mittelabschnitt (3) hergestellt wird und das Buffy-Coat (65) aus dem Mittelabschnitt (3) in den Einfrierbehälter (12) gelangt.

3. Verfahren nach Anspruch 1 oder 2, **dadurch gekennzeichnet, dass** dem Blut vor der Zentrifugierung eine weitere Substanz, insbesondere Hydroxyethylstärkelösung (HES), zugegeben wird.

4. Verfahren nach einem der Ansprüche 1 bis 3, **dadurch gekennzeichnet, dass** das Aufnahmevolumen des Einfrierbehälters (12) an die nach der Zentrifugierung sichtbare Menge an Buffy-Coat, ggf. unter Berücksichtigung der Zugabe mindestens einer weiteren Substanz, insbesondere Dimethylsulfoxid (DMSO), Blutplasma oder eine Mischung daraus, angepasst wird und diese Anpassung insbesondere durch die Abtrennung von Kompartimenten des Einfrierbehälters (12) erfolgt.

5. Verfahren nach einem der Ansprüche 1 bis 4, **dadurch gekennzeichnet, dass** die Befüllung des Abtrennbehälters (1) mit Blut über dessen steril konnektierbaren Anschluss (14) erfolgt, dass die Zugabe weiterer Substanzen in den Abtrennbehälter (1) oder in den Einfrierbehälter (12) über sterile Verbindungen erfolgt, dass der Abtrennbehälter (1), der Einfrierbehälter (12) und alle weiteren Entnahme- und / oder Zuführeinrichtungen mit Vorratsund/oder Misch- und/oder Transport- und/oder Dosierfunktion miteinander steril konnektiert sind und ein geschlossenes System ausbilden und dass alle Komponenten untereinander steril in Verbindung gebracht werden oder deren Verbindung steril unterbrochen werden oder die völlig voneinander steril abgetrennt werden.

6. Verfahren nach Anspruch 5, **dadurch gekennzeichnet, dass** eine Zuführeinrichtung (8) für Blut nach erfolgter Befüllung des Abtrennbehälters (1) und vor der Zentrifugierung steril diskonnektiert und entfernt wird.

7. Verfahren nach einem der Ansprüche 1 bis 6, **dadurch gekennzeichnet, dass** Entnahme- und / oder Zuführeinrichtungen (45) mit Vorrats- und/oder Misch- und/öder Transport- und/oder Dosierfunktion betreffend die Substanzen DMSO (26, 27), Blutplasma (30), ggf. HES (10), ggf. eine Druckausgleicheinrichtung (39), ggf. ein Aufnahmebehälter für Erythrozyten (49), mit dem Abtrennbehälter gemeinsam in einen Zentrifugenbecher (59) eingebracht werden und nach der Zentrifugierung nach und nach entfernt werden bis der Einfrierbehälter (12) übrig bleibt.

8. Verfahren nach Anspruch 7, **dadurch gekennzeichnet, dass** der übrig gebliebene präparierte Einfrierbehälter dann, ggf. unter Aufteilung in mindestens zwei Kompartimente, seiner Verwendung zugeführt wird.

9. Abtrennbehälter für eine Blutzentrifuge, insbesondere zur Durchführung des Verfahrens nach einem der Ansprüche 1 bis 8, mit einem Fußabschnitt (2) zur Aufnahme von Erythrozyten (64), mit einem Mittelabschnitt (3) zur Aufnahme von Buffy-Coat (65) und mit einem Kopfabschnitt (4) zur Aufnahme von Blutplasma (66), wobei der Mittelabschnitt (3) eine geringere Querschnittsabmessung aufweist als Kopf- und Fußabschnitt (2, 4), wobei die Abschnitte (2, 3, 4) strömungsverbunden sind und wobei der Fußabschnitt (2) vom Mittelabschnitt (3) abtrennbar ist, wobei der Fußabschnitt (2) bezüglich seines Aufnahmevolumens an das nach der Zentrifugierung zu erwartende Packvolumen der Erythrozyten (64) so anpassbar ist, dass das zu erwartende Packvolumen weitgehend vom Fußabschnitt (2) vollständig aufnehmbar ist und dass hierdurch die Phasengrenze (5) zwischen Erythrozyten (64) und Buffy-Coat (65) in einem zum Fußabschnitt (2) benachbarten Bereich des Mittelabschnittes (3) verläuft,
**dadurch gekennzeichnet,**
**dass** der Abtrennbehälter (1) einen Einfrierbehälter (12) umfasst und dass der Einfrierbehälter (12) den Mittelabschnittes (3) des Abtrennbehälters (1) integral umfasst und dass das Aufnahmevolumen des Einfrierbehälters (12) an die zu erwartende Ausbeute an zentrifugiertem Buffy-Coat (65) aus dem Mittelabschnitt (3), ggf. an die Zugabe mindestens einer weiteren Substanz, anpassbar ist.

10. Abtrennbehälter für eine Blutzentrifuge, insbesondere zur Durchführung des Verfahrens nach einem der Ansprüche 1 bis 8, mit einem Fußabschnitt (2) zur Aufnahme von Erythrozyten (64), mit einem Mittelabschnitt (3) zur Aufnahme von Buffy-Coat (65) und mit einem Kopfabschnitt (4) zur Aufnahme von Blutplasma (66), wobei der Mittelabschnitt (3) eine geringere Querschnittsabmessung aufweist als Kopf- und Fußabschnitt (2, 4), wobei die Abschnitte (2, 3, 4) strömungsverbunden sind und wobei der Fußabschnitt (2) vom Mittelabschnitt (3) abtrennbar ist, wobei der Fußabschnitt (2) bezüglich seines Aufnahmevolumens an das nach der Zentrifugierung zu erwartende Packvolumen der Erythrozyten (64) so anpassbar ist, dass das zu erwartende Packvolumen weitgehend vom Fußabschnitt (2) vollständig aufnehmbar ist und dass hierdurch die Phasengrenze (5) zwischen Erythrozyten (64) und Buffy-Coat (65) in einem zum Fußabschnitt (2) benachbarten Bereich des Mittelabschnittes (3) verläuft
**dadurch gekennzeichnet**
**dass** der Abtrennbehälter (1) einen Einfrierbehälter (12) umfasst, dass der Einfrierbehälter (12) dem Abtrennbehälter (1) zugeordnet und mit diesem strömungsverbindbar ist, dass der Fußabschnitt (2), der Mittelabschnitt (3) und der Kopfabschnitt (4) von Beginn an dreidimensional sind und dass der Einfrierbehälter (12) mit einer Zuführeinrichtung (26) für Dimethylsulfoxid - DMSO - und mit einer Spritze (25) steril verbunden ist, wobei die Spritze (25) Transport-, Vorrats- und/oder Mischfunktion wahrnimmt und dass der Abtrennbehälter (1), der Einfrierbehälter (12), die Zuführeinrichtung (26) für DMSO und die Spritze (25) Bestandteile eines geschlossenen Systems sind, wobei die Einzelkomponenten innerhalb von Behältnissen (45) enthalten sind, die im Sinne von sterilen Ummantelungen wirken und keinen Austausch mit der Umwelt zulassen und wobei die Einzelkomponenten steril konnektiert und steril abtrennbar sind.

11. Abtrennbehälter nach Anspruch 9 oder 10, **dadurch gekennzeichnet, dass** der Abtrennbehälter (1) einen Aufnahmebehälter (49) für Erythrozyten (64) aufweist, der insbesondere steril mit dem Anschluss (14) verbunden ist und von diesem nach der Zentrifugierung und nach der ggf. erforderlichen Aufnahme von Erythrozyten (64) insbesondere steril von diesem abtrennbar ist.

12. Abtrennbehälter Anspruch 10 oder 11, **dadurch gekennzeichnet, dass** das Aufnahmevolumen des Einfrierbehälters (12) an die zu erwartende Ausbeute an zentrifugiertem Buffy-Coat (65) aus dem Mittelabschnitt (3), ggf. an die Zugabe mindestens einer weiteren Substanz, anpassbar ist.

13. Abtrennbehälter nach einem der Ansprüche 9 bis 12, **dadurch gekennzeichnet, dass** der Abtrennbehälter (1) vor der Zentrifugierung mit einer Zuführeinrichtung (8) zur Zugabe des Blutes insbesondere steril und insbesondere über einen Anschluss (14) verbunden ist, wobei die Zuführeinrichtung (8) für Blut nach der Befüllung des Abtrennbehälters (1) mit Blut und vor der Zentrifugierung von diesem insbesondere steril abtrennbar ist.

14. Abtrennbehälter nach Anspruch 13, **dadurch gekennzeichnet, dass** der Abtrennbehälter (1) mit einer Zuführeinrichtung (10) für HES insbesondere steril und insbesondere über einen Anschluss (14, 38) verbunden ist, wobei die Zuführeinrichtung (10) für HES nach der Befüllung des Abtrennbehälters (1) mit HES von diesem insbesondere steril abtrennbar ist.

15. Abtrennbehälter nach einem der Ansprüche 9 bis 14, **dadurch gekennzeichnet, dass** der Abtrennbehälter (1) eine Druckausgleicheinrichtung (39, 46) für Luft öder Inertgas umfasst.

16. Abtrennbehälter nach einem der Ansprüche 9 bis 15, **dadurch gekennzeichnet, dass** die Zuführeinrichtung (8) einen Blutsammelbehälter (46) umfasst, der erforderlichenfalls auch als Druckausgleicheinrichtung fungiert, und dass eine Zuführeinrichtung (10) für HES insbesondere steril mit dem Blutsammelbehälter (46) verbunden ist, wobei die Zuführeinrichtung (10) für HES nach der Befüllung des Abtrennbehälters (1) mit HES über den Blutsammelbehälter (46) von diesem (46) insbesondere steril abtrennbar ist.

17. Abtrennbehälter nach einem der Ansprüche 9 bis 16, **dadurch gekennzeichnet, dass** zwischen der Zuführeinrichtung (26) für DMSO und der Spritze (25) vorzugsweise ein Rundfilter (50) vorgesehen ist.

18. Abtrennbehälter nach einem der Ansprüche 14 bis 17, **dadurch gekennzeichnet, dass** die Zuführeinrichtung (10) für HES, ggf. der Blutsammelbehälter (46) der Zuführeinrichtung (8) für Blut, Bestandteile eines geschlossenen Systems sind, wobei die Einzelkomponenten innerhalb von Behältnissen (45) enthalten sind, die im Sinne von sterilen Ummantelungen wirken und keinen Austausch mit der Umwelt zulassen und wobei die Einzelkomponenten steril konnektiert und steril abtrennbar sind.

19. Abtrennbehälter nach einem der Ansprüche 9 bis 18, **dadurch gekennzeichnet, dass** am Fuß-, Mittel- und Kopfabschnitt (2, 3, 4) des Abtrennbehälters (1), ggf. an einem Probenahmeabschnitt (47), mindestens eine Identifikationsnummer (48) aufgebracht ist.

20. Abtrennbehälter nach einem der Ansprüche 9 bis 19, **dadurch gekennzeichnet, dass** dass der Fußabschnitt (2), der Mittelabschnitt (3) und der Kopfabschnitt (4) des Abtrennbehälters (1) ein Einfüllvolumen von 500 ml aufweisen.

21. System zur Befüllung eines Einfrierbehälters mit einer nach Zentrifugierung gewonnenen Blutfraktion, nämlich Buffy-Coat, und zur Präparation des Buffy-Coats zum Zwecke der Kryokonservierung im Einfrierbehälter, unter Verwendung eines Abtrennbehälters nach einem der Ansprüche 9 und 11 bis 20 nicht in Verbindung mit Anspruch 10 und zur Durchführung des Verfahrens nach einem der Ansprüche 1 und 3 bis 8 nicht in Verbindung mit Anspruch 2,
wobei eine Zuführeinrichtung (8) für Blut, ein Abtrennbehälter (1) nach einem der Ansprüche 9 und 11 bis 20 nicht in Verbindung mit Anspruch 10 und ein Einfrierbehälter (12) vorgesehen sind,
wobei die Zuführeinrichtung (8) für Blut mindestens eine Antikoagulans-Zuführung (20) und einen Probenahmeabschnitt (47) umfasst und mit dem Abtrennbehälter (1) steril verbunden ist,
wobei die Zuführeinrichtung (8) für Blut nach der Befüllung des Abtrennbehälters (1) und vor der Zentrifugierung vom Abtrennbehälter (1) steril abtrennbar ist,
wobei der Abtrennbehälter (1) einen Fußabschnitt (2), einen Mittelabschnitt (3) und einen Kopfabschnitt (4) aufweist,
wobei der Mittelabschnitt (3) nach der Zentrifugierung unter Entfernung des Kopf- und Fußabschnittes (2, 4) zu einem Einfrierbehälter (12) transformierbar ist,
wobei der Mittelabschnitt (3) bzw. der Einfrierbehälter (12) mit einer ummantelten Spritze (25), einer Zuführeinrichtung (26) für DMSO und einer Druckausgleicheinrichtung (39) steril verbunden ist,
wobei die ummantelte Spritze (25), die Zuführeinrichtung (26) für DMSO und die Druckausgleicheinrichtung (39) nach der Präparation des Buffy-Coats (65) im Einfrierbehälter (12) von diesem (12) steril abtrennbar sind.

22. System nach Anspruch 21, **dadurch gekennzeichnet, dass** die Zuführeinrichtung (8) für Blut (55) einen Blutsammelbehälter (46) umfasst.

23. System nach Anspruch 21 oder 20, **dadurch gekennzeichnet, dass** nach Schließung des Blutsammelbehälters (46) oder des Abtrennbehälters (1) gegenüber der Zuführeinrichtuhg (8) für Blut ein geschlossenes System vorliegt.

24. System nach Anspruch 23, **dadurch gekennzeichnet, dass** das System eine Zuführeinrichtung (10) für HES umfasst, die mit dem Abtrennbehälter (1), ggf. über einen Blutsammelbehälter (46), steril verbunden ist und nach der Befüllung des Abtrennbehälters (1) mit HES von diesem steril abtrennbar ist.

## Claims

1. A method for separating blood, wherein different blood fractions, specifically erythrocytes, buffy coat, and blood plasma, are obtained,
wherein blood is introduced into a separation container and is then centrifuged into different, fluidically connected sections of the separation container arranged above one another, specifically a top section for receiving the blood plasma, a centre section for receiving the buffy coat, and a bottom section for receiving the erythrocytes,
• wherein the future packing volume of the erythrocytes to be centrifuged is determined using the haematocrit value of the supplied blood,
• wherein the capacity of the bottom section is adapted to the expected packing volume of the erythrocytes after centrifugation such that the phase boundary forming between buffy coat and erythrocytes during centrifugation is positioned in a region of the centre section of the separation container that is adjacent to the bottom section, and
• wherein the quantity of supplied blood is introduced into the separation container in an exact volume, taking into account the expected packing volume of the erythrocytes,
**characterised in that**
after centrifugation the bottom section (2) is separated from the centre section (3), and **in that** the centre section (3) is part of a freezer container (12) that during centrifugation is separated from the other region of the freezer container (12), and **in that** after centrifugation the connection to the previously separated region of the freezer container (12) is produced and the buffy coat travels at least into parts of the entire freezer container (12).

2. A method for separating blood, wherein different blood fractions, specifically erythrocytes, buffy coat, and blood plasma, are obtained,
wherein blood is introduced into a separation container and is then centrifuged into different, fluidically connected sections of the separation container arranged above one another, specifically a top section for receiving the blood plasma, a centre section for receiving the buffy coat, and a bottom section for receiving the erythrocytes,
• wherein the future packing volume of the erythrocytes to be centrifuged is determined using the haematocrit value of the supplied blood,
• wherein the capacity of the bottom section is adapted to the expected packing volume of the erythrocytes after centrifugation such that the phase boundary forming between buffy coat and erythrocytes during centrifugation is positioned in a region of the centre section of the separation container that is adjacent to the bottom section, and
• wherein the quantity of supplied blood is introduced into the separation container in an exact volume, taking into account the expected packing volume of the erythrocytes, **characterised in that**
after centrifugation the bottom section (2) is separated from the centre section (3), and **in that** after centrifugation and after separation of the bottom section from the centre section a fluidic connection is established between a freezer container (12) which is associated with the separation container (1) and may be fluidically connected thereto and the centre section (3), and the buffy coat (65) passes from the centre section (3) into the freezer container (12).

3. The method according to Claim 1 or 2, **characterised in that** another substance, in particular hydroxyethyl starch (HES) solution, is added to the blood prior to centrifugation.

4. The method according to one of Claims 1 to 3, **characterised in that** the capacity of the freezer container (12) is adapted to the visible quantity of buffy coat after centrifugation, where necessary taking into account the addition of at least one other substance, in particular dimethyl sulfoxide (DMSO), blood plasma, or a mixture thereof, and this adaptation occurs in particular by the separation of compartments of the freezer container (12).

5. The method according to one of Claims 1 to 4, **characterised in that** blood is introduced into the separation container (1) via the sterile connectable connector (14) of the separation container, **in that** the other substances are added to the separation container (1) or to the freezer container (12) via sterile connections, **in that** the separation container (1), the freezer container (12), and all other removal and/or supply devices having a reservoir and/or mixing and/or transport and/or metering function are connected to one another in a sterile manner and embody a closed system, and **in that** all components are connected to one another in a sterile manner or their connection is interrupted in a sterile manner and they are separated from one another in a completely sterile manner.

6. The method according to Claim 5, **characterised in that** a supply device (8) for blood is disconnected and removed in a sterile manner after the separation container (1) has been filled and before centrifugation.

7. The method according to one of Claims 1 to 6, **characterised in that** removal and/or supply devices (45) having a reservoir and/or mixing and/or transport and/or metering function for the substances DMSO (26, 27), blood plasma (30), where necessary HES (10), where necessary a pressure equalisation device (39), where necessary a receptacle for erythrocytes (49), are added with the separation container together to a centrifuge beaker (59) and after centrifugation are gradually removed until the freezer container (12) is left.

8. The method according to Claim 7, **characterised in that** the prepared freezer container that is left is then supplied for its use, where necessary being divided into at least two compartments.

9. A separation container for a blood centrifuge, in particular for carrying out the method according to one of Claims 1 to 8, having a bottom section (2) for receiving erythrocytes (64), having a centre section (3) for receiving buffy coat (65), and having a top section (4) for receiving blood plasma (66), wherein the centre section (3) has a smaller cross-sectional dimension than the top and bottom sections (2, 4), wherein the sections (2, 3, 4) are fluidically connected, and wherein the bottom section (2) is separable from the centre section (3), wherein the capacity of the bottom section (2) may be adapted to the expected packing volume of the erythrocytes (64) after centrifugation such that the expected packing volume may largely be completely accommodated by the bottom section (2) and such that through this the phase boundary (5) between erythrocytes (64) and buffy coat (65) runs in a region of the centre section (3) that is adjacent to the bottom section (2),
**characterised in that**
the separation container (1) includes a freezer container (12), and **in that** the freezer container (12) integrally includes the centre section (3) of the separation container (1), and **in that** the capacity of the freezer container (12) may be adapted to the expected yield of centrifuged buffy coat (65) from the centre section (3), where necessary to the addition of at least one other substance.

10. A separation container for a blood centrifuge, in particular for carrying out the method according to one of Claims 1 to 8, having a bottom section (2) for receiving erythrocytes (64), having a centre section (3) for receiving buffy coat (65), and having a top section (4) for receiving blood plasma (66), wherein the centre section (3) has a smaller cross-sectional dimension than the top and bottom sections (2, 4), wherein the sections (2, 3, 4) are fluidically connected, and wherein the bottom section (2) is separable from the centre section (3), wherein the capacity of the bottom section (2) may be adapted to the expected packing volume of the erythrocytes (64) after centrifugation such that the expected packing volume may essentially be completely accommodated by the bottom section (2) and such that through this the phase boundary (5) between erythrocytes (64) and buffy coat (65) runs in a region of the centre section (3) that is adjacent to the bottom section (2),
**characterised in that**
the separation container (1) includes a freezer container (12), **in that** the freezer container (12) is associated with the separation container (1) and may be fluidically connected thereto, **in that** the bottom section (2), the centre section (3) and the top section (4) are three-dimensional from the beginning, and **in that** the freezer container (12) is connected in a sterile manner to a supply device (26) for dimethyl sulfoxide (DMSO) and to a syringe (25), wherein the syringe (25) takes on a transport, storage and/or mixing function, and **in that** the separation container (1), the freezer container (12), the supply device (26) for DMSO and the syringe (25) are parts of a closed system, wherein the individual components are contained within containers (45), which act in the sense of sterile coverings and do not permit any exchange with the surrounding environment, and wherein the individual components are connected in a sterile manner and may be separated in a sterile manner.

11. The separation container according to Claim 9 or 10, **characterised in that** the separation container (1) has a receptacle (49) for erythrocytes (64) that is connected in particular in a sterile manner to the connector (14) and may be separated therefrom, in particular in a sterile manner, after centrifugation and after any required receiving of erythrocytes (64).

12. The separation container according to Claim 10 or 11, **characterised in that** the capacity of the freezer container (12) may be adapted to the expected yield of centrifuged buffy coat (65) from the centre section (3) and where necessary to the addition of at least one other substance.

13. The separation container according to one of Claims 9 to 12, **characterised in that** the separation container (1) prior to centrifuging is connected to a supply device (8) for supplying the blood, in particular in a sterile manner, and in particular via a connector (14), wherein, after the separation container (1) has been filled with blood and before centrifugation, the supply device (8) for blood may be separated from the separation container, in particular in a sterile manner.

14. The separation container according to Claim 13, **characterised in that** the separation container (1) is connected to a supply device (10) for HES, in particular in a sterile manner, and in particular via a connector (14, 38), wherein, after the separation container (1) has been filled with HES, the supply device (10) for HES may be separated from the separation container, in particular in a sterile manner.

15. The separation container according to one of Claims 9 to 14, **characterised in that** the separation container (1) includes a pressure equalisation device (39, 46) for air or inert gas.

16. The separation container according to one of Claims 9 to 15, **characterised in that** the supply device (8) includes a blood collection container (46) that as necessary also functions as a pressure equalisation device, and **in that** a supply device (10) for HES is connected to the blood collection container (46), in particular in a sterile manner, wherein, after the separation container (1) has been filled with HES via the blood collection container (46), the supply device (10) for HES may be separated from the blood collection container (46), in particular in a sterile manner.

17. The separation container according to one of Claims 9 to 16, **characterised in that** a preferably round filter (50) is provided between the supply device (26) for DMSO and the syringe (25).

18. The separation container according to one of Claims 14 to 17, **characterised in that** the supply device (10) for HES, where necessary the blood collection container (46) of the supply device (8) for blood, are components of a closed system, wherein the individual components are included inside containers (45) that act in the sense of sterile coverings and do not permit any exchange with the surrounding environment, and wherein the individual components are connected in a sterile manner and may be separated in a sterile manner.

19. The separation container according to one of Claims 9 to 18, **characterised in that** at least one identification number (48) is applied to the bottom, centre, and top sections (2, 3, 4) of the separation container (1), where necessary to a sampling section (47).

20. The separation container according to one of Claims 9 to 19, **characterised in that** the bottom section (2), the centre section (3) and the top section (4) of the separation container (1) have a fill volume of 500 ml.

21. A system for filling a freezer container with a blood fraction obtained after centrifugation, specifically buffy coat, and for preparing the buffy coat for the purposes of cryopreservation in the freezer container, using a separation container according to one of Claims 9 and 11 to 20 not in conjunction with Claim 10 and for carrying out the method according to one of Claims 1 and 3 to 8 not in conjunction with Claim 2,
wherein a supply device (8) for blood, a separation container (1) according to one of Claims 9 and 11 to 20 not in conjunction with Claim 10, and a freezer container (12) are provided,
wherein the supply device (8) for blood includes at least one anticoagulant supply (20) and one sampling section (47) and is connected in a sterile manner to the separation container (1),
wherein the supply device (8) for blood may be separated in a sterile manner from the separation container (1) after the separation container (1) has been filled and before centrifugation,
wherein the separation container (1) has a bottom section (2), a centre section (3), and a top section (4),
wherein the centre section (3) may be transformed into a freezer container (12) after centrifugation, with the top and bottom sections (2, 4) removed,
wherein the centre section (3) or the freezer container (12) is connected in a sterile manner to a covered syringe (25), a supply device (26) for DMSO, and a pressure equalisation device (39),
wherein the covered syringe (25), the supply device (26) for DMSO, and the pressure equalisation device (39) may be separated in a sterile manner from the freezer container (12) after the buffy coat (65) has been prepared in the freezer container (12).

22. The system according to Claim 21, **characterised in that** the supply device (8) for blood (55) includes a blood collection container (46).

23. The system according to Claim 21 or 20, **characterised in that**, after closing off the blood collection container (46) or the separation container (1) from the supply device (8) for blood, a closed system is provided.

24. The system according to Claim 23, **characterised in that** the system includes a supply device (10) for HES that is connected in a sterile manner to the separation container (1), where necessary via a blood collection container (46), and that after the separation container (1) has been filled with HES may be separated therefrom in a sterile manner.

## Revendications

1. Procédé de séparation du sang, dans lequel différentes fractions sanguines, à savoir érythrocytes, couche leuco-plaquettaire et plasma sanguin peuvent être obtenues,
sachant que du sang est rempli dans un récipient de séparation puis centrifugé dans différentes sections du récipient de séparation disposées les unes au-dessus des autres et en liaison d'écoulement, à savoir la section supérieure pour collecter le plasma sanguin, la section centrale pour collecter la couche leuco-plaquettaire et la section inférieure pour collecter les érythrocytes,
• sachant que le volume futur des érythrocytes à centrifuger est déterminé à l'aide de la valeur d'hématocrite du sang amené,
• sachant que le volume collecteur de la section inférieure est ainsi adapté au volume des érythrocytes prévu après la centrifugation que la limite de phase se formant lors de la centrifugation entre la couche leuco-plaquettaire et les érythrocytes est positionnée dans une partie de la section centrale du récipient de séparation adjacente à la section inférieure et
• sachant que la quantité de sang amené est indiquée avec un volume précis dans le récipient de séparation en tenant compte du volume d'érythrocytes prévu,
**caractérisé en ce que**
la séparation de la section inférieure (2) de la section centrale (3) a lieu après la centrifugation, et que la section centrale (3) fait partie d'un récipient de congélation (12) qui est séparé de la partie restante du récipient de congélation (12) pendant la centrifugation, et que la liaison vers la partie du récipient de congélation (12) séparée auparavant est établie après la centrifugation et la couche leuco-plaquettaire parvient au moins dans des parties de la totalité du récipient de congélation (12).

2. Procédé de séparation du sang, dans lequel différentes fractions sanguines, à savoir érythrocytes, couche leuco-plaquettaire et plasma sanguin peuvent être obtenues,
sachant que du sang est rempli dans un récipient de séparation puis centrifugé dans différentes sections du récipient de séparation disposées les unes au-dessus des autres et en liaison d'écoulement, à savoir la section supérieure pour collecter le plasma sanguin, la section centrale pour collecter la couche leuco-plaquettaire et la section inférieure pour collecter les érythrocytes,
• sachant que le volume futur des érythrocytes à centrifuger est déterminé à l'aide de la valeur d'hématocrite du sang amené,
• sachant que le volume collecteur de la section inférieure est ainsi adapté au volume des érythrocytes prévu après la centrifugation que la limite de phase se formant lors de la centrifugation entre la couche leuco-plaquettaire et les érythrocytes est positionnée dans une partie de la section centrale du récipient de séparation adjacente à la section inférieure et
• sachant que la quantité de sang amené est indiquée avec un volume précis dans le récipient de séparation en tenant compte du volume d'érythrocytes prévu,
**caractérisé en ce que**
la séparation de la section inférieure (2) de la section centrale (3) a lieu après la centrifugation, et qu'après la centrifugation et après la séparation de la section inférieure de la section centrale, une liaison d'écoulement entre un récipient de congélation (12) attribué au réservoir de séparation (1) et pouvant être mis en liaison d'écoulement avec celui-ci et la section centrale (3) est établie et la couche leuco-plaquettaire (65) parvient de la section centrale (3) au récipient de congélation (12).

3. Procédé selon la revendication 1 ou 2, **caractérisé en ce qu'**une substance supplémentaire, en particulier une solution d'amidon hydroxyéthylé (HES), est ajoutée au sang avant la centrifugation.

4. Procédé selon l'une des revendications 1 à 3, **caractérisé en ce que** le volume collecteur du récipient de congélation (12) est adapté à la quantité visible de couche leuco-plaquettaire après centrifugation, le cas échéant en tenant compte de l'ajout d'au moins une substance supplémentaire, en particulier de diméthylsulfoxyde (DMSO), de plasma sanguin ou un mélange de ceux-ci, et cette adaptation est effectuée en particulier par séparation de compartiments du récipient de congélation (12).

5. Procédé selon l'une des revendications 1 à 4, **caractérisé en ce que** le remplissage du réservoir de séparation (1) avec du sang est effectué par son raccordement (14) pouvant être raccordé de façon stérile, que l'ajout d'autres substances dans le récipient de séparation (1) ou dans le récipient de congélation (12) s'effectue par des liaisons stériles, que le récipient de séparation (1), le récipient de congélation (12) et tous les autres dispositifs de prélèvement et/ou d'alimentation avec fonction de stockage et/ou de mélange et/ou de transport et/ou de dosage sont connectés de façon stérile entre eux et forment un système fermé et que toutes les composantes peuvent être mises en liaison stérile entre elles ou que leur liaison peut être interrompue de façon stérile ou qu'elles peuvent être séparées totalement les unes des autres de façon stérile.

6. Procédé selon la revendication 5, **caractérisé en ce qu'**un dispositif d'alimentation (8) pour le sang est déconnecté et éloigné de façon stérile, une fois le remplissage du récipient de séparation (1) effectué et avant la centrifugation.

7. Procédé selon l'une des revendications 1 à 6, **caractérisé en ce que** des dispositifs de prélèvement et/ou d'alimentation (45) avec fonction de stockage et/ou de mélange et/ou de transport et/ou de dosage concernant les substances DMSO (26, 27), plasma sanguin (30), le cas échéant HES (10), le cas échéant un dispositif de compensation de pression (39) et le cas échéant un récipient collecteur pour les érythrocytes (49), peuvent être intégrés conjointement avec le récipient de séparation dans un bécher de centrifugation (59), puis enlevés petit à petit après la centrifugation jusqu'à ce qu'il ne reste plus que le récipient de congélation (12).

8. Procédé selon la revendication 7, **caractérisé en ce que** le récipient de congélation préparé restant est amené à utilisation ensuite, le cas échéant en le partageant en au moins deux compartiments.

9. Récipient de séparation pour une centrifugeuse de sang, en particulier destinée à exécuter le procédé selon l'une des revendications 1 à 8, comprenant une section inférieure (2) pour collecter des érythrocytes (64), une section centrale (3) pour collecter la couche leuco-plaquettaire (65) et une section supérieure (4) pour collecter du plasma sanguin (66), sachant que la section centrale (3) présente une dimension de section transversale inférieure aux sections supérieure et inférieure (2, 4), sachant que les sections (2, 3, 4) sont en liaison d'écoulement et sachant que la section inférieure (2) peut être séparée de la section centrale (3), sachant que la section inférieure (2), au vu de son volume de collecte, peut être adaptée de telle sorte au volume d'érythrocytes (64) prévu après la centrifugation que le volume prévu peut être collecté dans une grande mesure complètement par la section inférieure (2) et qu'ainsi, la limite de phase (5) entre les érythrocytes (64) et la couche leuco-plaquettaire (65) passe dans une partie de la section centrale (3) adjacente à la section inférieure (2),
**caractérisé en ce que**
le récipient de séparation (1) contient un récipient de congélation (12) et que le récipient de congélation (12) inclut la section centrale (3) du récipient de séparation (1) en totalité et que le volume de collecte du récipient de congélation (12) peut être adapté au rendement prévu de couche leuco-plaquettaire (65) centrifugée de la section centrale (3), le cas échéant à l'ajout d'au moins une substance supplémentaire.

10. Récipient de séparation pour une centrifugeuse de sang, en particulier destinée à exécuter le procédé selon l'une des revendications 1 à 8, comprenant une section inférieure (2) pour collecter des érythrocytes (64), une section centrale (3) pour collecter la couche leuco-plaquettaire (65) et une section supérieure (4) pour collecter du plasma sanguin (66), sachant que la section centrale (3) présente une dimension de section transversale inférieure aux sections supérieure et inférieure (2, 4), sachant que les sections (2, 3, 4) sont en liaison d'écoulement et sachant que la section inférieure (2) peut être séparée de la section centrale (3), sachant que la section inférieure (2), au vu de son volume de collecte, peut être adaptée de telle sorte au volume d'érythrocytes (64) prévu après la centrifugation que le volume prévu peut être collecté dans une grande mesure complètement par la section inférieure (2) et qu'ainsi, la limite de phase (5) entre les érythrocytes (64) et la couche leuco-plaquettaire (65) passe dans une partie de la section centrale (3) adjacente à la section inférieure (2),
**caractérisé en ce que**
le récipient de séparation (1) comprend un récipient de congélation (12), que le récipient de congélation (12) est attribué au récipient de séparation (1) et peut être en liaison d'écoulement avec celui-ci, que la section inférieure (2), la section centrale (3) et la section supérieure (4) sont tridimensionnelles dès le départ et que le récipient de congélation (12) est relié de façon stérile à un dispositif d'alimentation (26) de diméthylsulfoxyde - DMSO et à une seringue (25), sachant que la seringue (25) a une fonction de transport, stockage et/ou mélange, et que le récipient de séparation (1), le récipient de congélation (12), le dispositif d'alimentation (26) du DMSO et la seringue (25) font partie d'un système fermé, sachant que les composantes individuelles sont contenues à l'intérieur de contenants (45) qui agissent au sens d'enveloppes stériles et ne permettent aucun échange avec l'environnement, et sachant que les composantes individuelles sont connectées de façon stériles et séparables de façon stérile.

11. Récipient de séparation selon la revendication 9 ou 10, **caractérisé en ce que** le récipient de séparation (1) présente un récipient de collecte (49) pour des érythrocytes (64) qui est en particulier relié de façon stérile au raccordement (14) et peut être séparé de celui-ci en particulier de façon stérile après la centrifugation et après la collecte nécessaire des érythrocytes (64).

12. Récipient de séparation 10 ou 11, **caractérisé en ce que** le volume de collecte du récipient de congélation (12) peut être adapté au rendement prévu de couche leuco-plaquettaire (65) centrifugée de la section centrale (3), le cas échéant à l'ajout d'au moins une substance supplémentaire.

13. Récipient de séparation selon l'une des revendications 9 à 12, **caractérisé en ce que** le récipient de séparation (1), avant la centrifugation, est relié en particulier de façon stérile et en particulier par un raccordement (14) à un dispositif d'alimentation (8) pour ajouter le sang, sachant que le dispositif d'alimentation (8) pour le sang peut être séparé de celui-ci en particulier après le remplissage du récipient de séparation (1) avec du sang et avant la centrifugation.

14. Récipient de séparation 13, **caractérisé en ce que** le récipient de séparation (1) est relié en particulier de façon stérile et en particulier par un raccordement (14, 38) à un dispositif d'alimentation (10) pour HES, sachant que le dispositif d'alimentation (10) pour HES peut être séparé de celui-ci en particulier de façon stérile après le remplissage du récipient de séparation (1) avec du HES.

15. Récipient de séparation selon l'une des revendications 9 à 14, **caractérisé en ce que** le récipient de séparation (1) comprend un dispositif de compensation de pression (39, 46) pour de l'air ou du gaz inerte.

16. Récipient de séparation selon l'une des revendications 9 à 15, **caractérisé en ce que** le dispositif d'alimentation (8) comprend un récipient de collecte de sang (46) qui sert également de dispositif de compensation de pression si nécessaire, et que le dispositif d'alimentation (10) pour HES est relié en particulier de façon stérile au récipient de collecte de sang (46), sachant que le dispositif d'alimentation (10) pour HES peut être séparé en particulier de façon stérile du dispositif de compensation de pression (46) par celui-ci (46) après le remplissage du récipient de séparation (1) avec du HES.

17. Récipient de séparation selon l'une des revendications 9 à 16, **caractérisé en ce qu'**un filtre rond (50) est prévu de préférence entre le dispositif d'alimentation (26) pour DMSO et la seringue (25).

18. Récipient de séparation selon l'une des revendications 14 à 17, **caractérisé en ce que** le dispositif d'alimentation (10) pour HES, le cas échéant le dispositif de compensation de pression (46) du dispositif d'alimentation (8) pour le sang, sont des composantes d'un système fermé, sachant que les composantes individuelles sont contenues à l'intérieur de récipients (45) qui agissent au sens d'enveloppes stériles et ne permettent aucun échange avec l'environnement, et sachant que les composantes individuelles sont reliées de façon stérile et peuvent être séparées de façon stérile.

19. Récipient de séparation selon l'une des revendications 9 à 18, **caractérisé en ce qu'**au moins un numéro d'identification (48) est apposé sur les sections inférieure, centrale et supérieure (2, 3, 4) du récipient de séparation (1), cas échéant sur une section de prélèvement d'échantillon (47).

20. Récipient de séparation selon l'une des revendications 9 à 19, **caractérisé en ce que** la section inférieure (2), la section centrale (3) et la section supérieure (4) du récipient de séparation (1) présentent un volume de remplissage de 500 ml.

21. Système de remplissage d'un récipient de congélation avec une fraction sanguine obtenue après centrifugation, à savoir couche leuco-plaquettaire, et pour préparer la couche leuco-plaquettaire dans le but d'une cryoconservation dans le récipient de congélation, en utilisant un récipient de séparation selon l'une des revendications 9 et 11 à 20 sans liaison avec la revendication 10 et pour exécuter le procédé selon l'une des revendications 1 et 3 à 8 sans liaison avec la revendication 2,
sachant qu'un dispositif d'alimentation (8) pour le sang, un récipient de séparation (1) selon l'une des revendications 9 et 11 à 20 sans liaison avec la revendication 10 et un récipient de congélation (12) sont prévus,
sachant que le dispositif d'alimentation (8) pour le sang comprend au moins une amenée d'anticoagulant (20) et une section de prélèvement d'échantillon (47) et est relié de façon stérile au récipient de séparation (1),
sachant que le dispositif d'alimentation (8) pour le sang peut être séparé de façon stérile du récipient de séparation (1) après le remplissage du récipient de séparation (1) et avant la centrifugation,
sachant que le récipient de séparation (1) présente une section inférieure (2), une section centrale (3) et une section supérieure (4),
sachant que la section centrale (3) peut être transformée en un récipient de congélation (12) après la centrifugation, en éloignant les sections supérieure et inférieure (2, 4),
sachant que la section centrale (3), respectivement le récipient de congélation (12) est relié de façon stérile à une seringue (25) gainée, un dispositif d'alimentation (26) pour DMSO et un dispositif de compensation de pression (39),
sachant que la seringue (25) gainée, le dispositif d'alimentation (26) pour DMSO et le dispositif de compensation de pression (39) peuvent être séparés de façon stérile du récipient de congélation (12) après préparation de la couche leuco-plaquettaire (65) dans celui-ci (12).

22. Système selon la revendication 21, **caractérisé en ce que** le dispositif d'alimentation (8) pour le sang (55) comprend un récipient de collecte de sang (46).

23. Système selon la revendication 21 ou 20, **caractérisé en ce qu'**un système fermé est présent après fermeture du récipient de collecte de sang (46) ou du récipient de séparation (1) contre le dispositif d'alimentation (8) pour le sang.

24. Système selon la revendication 23, **caractérisé en ce que** le système comprend un dispositif d'alimentation (10) pour HES, qui est relié de façon stérile au récipient de séparation (1), le cas échéant par un récipient de collecte de sang (46) et est séparé de celui-ci de façon stérile après le remplissage du récipient de séparation (1) en HES.
